# EUROPEAN PATENT SPECIFICATION

(11) **EP 3 076 362 B1**
(45) Date of publication and mention of the grant of the patent: **11.03.2020**
(21) Application number: 14865707.5
(22) Date of filing: 28.11.2014
(51) Int. Cl.: G16H 10/60

(54) **MEDICAL INFORMATION PROCESSING DEVICE, METHOD, AND PROGRAM**
VORRICHTUNG, VERFAHREN UND PROGRAMM ZUR VERARBEITUNG VON MEDIZINISCHEN INFORMATIONEN
DISPOSITIF, PROCÉDÉ ET PROGRAMME DE TRAITEMENT D'INFORMATIONS MÉDICALES

(30) Priority: 28.11.2013 JP 2013245954
(43) Date of publication of application: 05.10.2016
(73) Proprietor: FUJIFILM Corporation, Tokyo 106-8620 (JP)
(72) Inventor: KANADA, Shoji, Ashigarakami-gun Kanagawa 258-8538 (JP)
(74) Representative: Klunker IP Patentanwälte PartG mbB
(86) International application number: PCT/JP2014/005960
(87) International publication number: WO 2015/079705

(56) References cited:
- EP-A2- 0 483 595
- WO-A1-2011/039741
- JP-A- 2007 108 812
- JP-A- 2011 150 395
- US-A1- 2005 154 627

## Description

### BACKGROUND OF THE INVENTION

### 1. Field of the Invention

The present invention relates to a medical information processing device and method for analyzing and processing medical information of a subject patient, and a computer-readable recording medium in which a program for analyzing and processing the medical information of a subject patient is stored.

### 2. Description of the Related Art

In recent years, in the medical field, techniques for using various kinds of medical information, which are acquired for medical care of patients, for diagnostic assistance have been drawing attention. For example, WO2012/001920A and WO2012/001921A relate to a technique of extracting the side effects of drugs, and disclose a technique of extracting information indicating an abnormality, such as a sudden change in the amount of a specific drug, from stored past information of a patient and detecting information, which may be highly relevant to the side effects of the drug, from the extracted information indicating an abnormality.

In addition, for reference information for the determination of a disease name or the determination of treatment policy or the like, it is predicted there will be techniques for assisting the understanding of the symptoms of the subject patient based on various kinds of inspection data. Diseases include acute diseases, which rapidly occur and are short-term diseases, and chronic diseases, which are difficult to cure and are long-term diseases. In the case of a chronic disease, there is a demand to check changes in the symptoms of a patient by observing the progress over a long period of time, such as several months.

EP 0 483 595 A2 discloses a medical information processing device having a dosage detail acquisition unit that acquires a plurality of dosage details in which information specifying a drug administered to a subject patient, a drug amount and a dosing date are associated with each other. A determination unit identifies a dosing period among dosing periods in which the administered drug and the drug amount are substantially the same. A dosing period is specified as a stable dosing period in which dosage details are common.

### SUMMARY OF THE INVENTION

In the case of a chronic disease, treatment to maintain the disease in a stable state is performed while checking whether the symptoms of the patient have been exacerbated from various kinds of inspection data. However, in a case where the patient is suffering from a chronic disease, for an inspection item used to determine the symptoms of the patient, inspection data indicating abnormalities continues over a medium or a long period of time. Accordingly, forms in which abnormalities of inspection data are exhibited also change with a patient. For this reason, in many cases, it is difficult to determine the exacerbation of the symptoms of the patient suffering from a chronic disease using a method of determining inspection data by comparison with a determined reference value. In a case where the patient is suffering from other diseases in addition to the chronic disease, inspection data may be changed due to the influence of other diseases. Therefore, it is required to determine the exacerbation of the symptoms of the subject patient from inspection data from which the influence of other diseases has been eliminated.

Under such circumstances, when determining the symptoms of a chronic disease, doctors comprehensively determine previous diseases of the subject patient by reviewing medical information, such as medical records, thereby determining whether the symptoms of the chronic disease of the patient are stable or are being exacerbated. If the task of checking the symptoms of a chronic disease is supported by such inspection data or medical information of the subject patient, this is believed to be beneficial for improving diagnostic efficiency and diagnostic accuracy.

Focusing on the fact that treatment to maintain the symptoms of a chronic disease in a stable state is continued for a subject patient suffering from a chronic disease, the present inventors have found by intensive studies that it is possible to provide information, which is useful for doctors to understand the symptoms of the subject patient, by specifying and providing a period, for which treatment to maintain the symptoms of the subject patient in a stable state is continued, in order to check the symptoms of such a chronic disease.

In view of the aforementioned situation, it is an object of the present invention to provide a medical information processing device, method, and program capable of estimating and acquiring a stable dosing period for which treatment to maintain a stable state for the symptoms of a patient is performed for a chronic disease.

In view of the above, a medical information processing device of the present invention comprises the features of claim 1, a medical information processing method executed by this device is defined by claim 22.

A medical information display control program of the present invention causes a computer to execute the method described above.

The "information specifying the drug" includes all pieces of information that can specify the type of drug. As an example, the information specifying the drug may be a therapeutic category number, or may be a drug code, or may be a drug name.

The "amount of drug" means the amount of drug administered to a patient in a predetermined unit of time. For example, it is preferable that the amount of drug represents the amount of drug on a daily basis. In the case of administering the drug multiple times in a day, the total amount of administration in a day can be set as the amount of drug. In the case of one administration in a week, the amount of drug administered by one administration may be averaged on a daily basis and the result may be set as the amount of drug, or the amount of drug on a weekly basis may be set as the amount of drug.

The "dosing date of the drug" means a day on which the drug is taken by being administered to the patient. For example, in the case of a drug administered by injection, the date of injection of the drug can be set as the dosing date of the drug. In the case of a prescribed drug, it is possible to specify the amount of drug to be taken per day and the taking date based on information or the like at the time of formulation and set the taking date as the dosing date of the drug.

The "dosing period of the drug for which it can be regarded that both the information specifying the drug and the amount of drug are the same" is preferably a period for which it can be regarded that both the information specifying the drug and the dose of the drug are substantially the same. In a case where treatment to maintain a stable state for a chronic disease is performed on the patient, a specific drug is applied to the patient over a period equal to or longer than a predetermined period while maintaining a fixed amount of drug in many cases. Therefore, by specifying a period for which both the information specifying the drug and the dose of the drug are the same, it is possible to specify the implementation period of treatment to maintain a stable state for a chronic disease. Here, in a case where it can be regarded that the information specifying substantially the same drug and the dose of the drug are maintained and managed for the patient by the doctor, it is regarded that both the information specifying the drug and the dose of the drug are the same. For example, for a case where a period for which the patient forgets to take a prescribed medicine temporarily occurs or a case where an interval at which the patient is subjected to medical examination is extended and taking the prescribed medicine ends and accordingly a no-dosing period temporarily occurs, it can be regarded that both the information specifying the drug and the dose of the drug are substantially the same. In addition, there may be a case where doctors finely adjust the amount of drug while observing the condition of the patient. Accordingly, for a change in the amount of drug equal to or less than a predetermined threshold value, it can be regarded that both the information specifying the drug and the dose of the drug are substantially the same. In addition, also for a case where the original drug is replaced with a generic drug, it can be regarded that both the information specifying the drug and the dose of the drug are substantially the same. The information specifying substantially the same drug may indicate a common action of drug (therapeutic category number).

For the "dosing period equal to or longer than a predetermined period among dosing periods of the drug for which it can be regarded that both the information specifying the drug and the amount of drug are the same", a period for which it can be estimated that treatment to maintain a stable state for a chronic disease is performed can be set as the predetermined period. For example, the predetermined period can be set to several weeks to several months or more.

The "stable dosing period" means a period excluding at least one of a predetermined initial period and a predetermined final period from the dosage details common period. In the dosage details common period for maintaining a stable state for a chronic disease, in the predetermined initial period, there is a possibility that changes in the symptoms due to a change in the treatment details from the treatment before medication will occur. In addition, in the predetermined final period, there is a possibility that changes in the symptoms will occur, for example, since the dosage details common period ends due to a change in the dosage details in the dosage details common period. Therefore, by setting the period excluding at least one of the predetermined initial period and the predetermined final period as a stable dosing period, it is possible to appropriately use the stable dosing period as a period for checking whether or not there is a stable state for the chronic disease. In addition, as the stable dosing period, it is preferable to set an appropriate period, for which it is possible to extract the features of inspection data, based on the findings in the medical diagnosis. For example, it is preferable to set an appropriate period as a stable dosing period for each disease or each determination purpose. In addition, the "predetermined initial period" is a period excluding a period in which it is considered that changes in the symptoms due to a change in the treatment details from the treatment before medication occur, and may be a period of one or more days set in advance. It is preferable that the "predetermined initial period" is appropriately set in accordance with the details required for the disease of the patient or the dosage details common period. In addition, the "predetermined final period" may be a period excluding a period in which it is considered that changes in the symptoms occur, for example, since the dosage details common period ends due to a change in the dosage details in the dosage details common period, and may be a period of one or more days set in advance. It is preferable that the "predetermined final period" is appropriately set in accordance with the details required for the disease of the patient or the dosage details common period.

In the medical information processing device according to the present invention, it is preferable that the dosage details acquisition unit acquires a disease of the subject patient as the target disease and selects and acquires the dosage details corresponding to a disease associated drug, which is known to be administered for the target disease, among the acquired dosage details of the subject patient.

The "inspection time of inspection data" may be expressed in any unit, such as a month, a week, a day, or a time, or may be a time substantially indicating whether or not the inspection data is data showing the condition of the patient in a certain period in time. In a blood test or the like, the "inspection time of inspection data" is preferably a time at which a sample (blood) is collected. In a case where the time of inspection is not clear, a predetermined time set arbitrarily for each inspection item, such as the acquisition time of inspection data or a time substantially regarded as the acquisition time of inspection data, may be the inspection time. For example, a time at which inspection data has been input (or acquired) first or a time at which inspection data has been updated last may be used as the inspection time.

In the medical information processing device according to the present invention, it is preferable that the feature amount analysis unit acquires determination information, in which a disease, a first inspection item that is a predetermined inspection item corresponding to the disease, feature amount specifying information that is information specifying the feature amount from the inspection data corresponding to the first inspection item, and the determination conditions corresponding to this feature amount specifying information are associated with each other, and acquires the feature amount according to the feature amount specifying information corresponding to the disease of the subject patient based on the determination information and that the determination section determines presence or absence of an abnormality at the determination time according to the determination conditions corresponding to the feature amount specifying information based on the acquired determination information.

As the "feature amount", any feature amount may be used as long as the feature amount can be extracted from the inspection data of the stable dosing period and the presence or absence of an abnormality at the determination time can be determined.

In addition, as "inspection items" used for the calculation of each feature amount, one or more inspection items of medical treatment items included in the target medical information may be arbitrarily set as long as it is possible to calculate a desired feature amount.

In the medical information processing device according to the present invention, it is preferable to further include a first display control unit that displays at least a piece of information included in the determination information corresponding to the determination, on a display screen, in a case where it is determined that there is an abnormality at the determination time based on the determination information.

In the medical information processing device according to the present invention, the stable dosing period determination unit can determine a first stable dosing period immediately before the determination time among first stable dosing periods that are the stable dosing periods before the determination time. The feature amount analysis unit can acquire a comparison feature amount, which is a feature amount determined from the specific inspection data of the first stable dosing period immediately before the determination time, and a first determination feature amount, which is a feature amount corresponding to the comparison feature amount determined from the inspection data of the subject patient at the determination time. The determination section can include a first determination section that determines whether or not first determination conditions for determining presence or absence of an abnormality at the determination time based on the comparison feature amount and the first determination feature amount are satisfied.

In the medical information processing device according to the present invention, it is preferable that the stable dosing period determination unit further determines a period having a start time after a first reference time that is a predetermined period before the determination time and having an end time before a second reference time that is after the start time and before an additional predetermined period from the determination time, among the first stable dosing periods immediately before the determination time, as a comparison stable dosing period and that the feature amount analysis unit determines the comparison feature amount from the specific inspection data included in the comparison stable dosing period.

In the above case, it is preferable that the stable dosing period determination unit acquires period specifying information in which a disease and the first reference time and the second reference time are associated with each other, acquires the first reference time and the second reference time corresponding to a target disease, which is a disease of the subject patient, based on the period specifying information, and determines the comparison stable dosing period based on the first reference time and the second reference time.

It is preferable that the "first reference time" and the "second reference time" are arbitrarily set so that the stable dosing period becomes a period, for which it is possible to extract the features of inspection data, based on the findings in the medical diagnosis. For example, it is preferable to set appropriate first and second reference times for each disease or each determination purpose.

In addition, the feature amount analysis unit may acquire the comparison feature amount using a predetermined number of pieces of the specific inspection data going back into a past in time series from an end of the first stable dosing period immediately before the determination time.

In the present invention, the comparison feature amount may be an average value of a plurality of pieces of the specific inspection data in the first stable dosing period corresponding to a second inspection item that is a predetermined inspection item.

In the present invention, the comparison feature amount may be a value of an approximate curve corresponding to the determination time in the approximate curve calculated from a plurality of pieces of the specific inspection data in the first stable dosing period corresponding to a third inspection item that is a predetermined inspection item.

In the present invention, the first determination conditions may be conditions in which it is determined that there is an abnormality at the determination time in a case where a difference between the comparison feature amount and the first determination feature amount or a ratio between the comparison feature amount and the first determination feature amount does not satisfy first threshold value conditions.

In the present invention, in the first threshold value conditions, a comparison feature amount for statistics that is a feature amount corresponding to the comparison feature amount and a first determination feature amount for statistics corresponding to the first determination feature amount may be calculated using a plurality of pieces of inspection data of a plurality of comparison subjects in the past, a plurality of determination parameters for statistics corresponding to a determination parameter for which threshold value determination in the first determination conditions is performed may be calculated using the calculated comparison feature amount for statistics and first determination feature amount for statistics, and a value away from the average value of the plurality of calculated determination parameters for statistics by a value equal to or greater than the variation range of the determination parameters for statistics may be set as a threshold value.

The "comparison feature amount for statistics corresponding to the comparison feature amount" means a feature amount that is specified by the same method as for the comparison feature amount using the inspection data of the same inspection item as for the comparison feature amount among a plurality of pieces of inspection data of a plurality of comparison subjects in the past. The "first determination feature amount for statistics corresponding to the first determination feature amount" means a feature amount that is specified by the same method as for the first determination feature amount using the inspection data of the same inspection item as for the first determination feature amount among a plurality of pieces of inspection data of a plurality of comparison subjects in the past.

In the medical information processing device according to the present invention, in a case where a value of the specific inspection data in the first stable dosing period is in a predetermined normal range, the feature amount analysis unit may calculate the comparison feature amount by replacing the value of the specific inspection data with an upper limit or a lower limit of the normal range.

In the medical information processing device according to the present invention, it is preferable that the stable dosing period determination unit determines a second stable dosing period that is the stable dosing period including the determination time in a final stage, the feature amount analysis unit acquires a second determination feature amount from a plurality of pieces of the inspection data of the subject patient in the second stable dosing period, and the determination section further includes a second determination section that determines whether or not second determination conditions for determining presence or absence of an abnormality at the determination time based on the second determination feature amount are satisfied.

In this case, the second determination feature amount may be a coefficient of an approximate curve calculated from a plurality of pieces of the specific inspection data in the second stable dosing period corresponding to a fourth inspection item that is a predetermined inspection item.

In the present invention, the second determination conditions may be conditions in which it is determined that there is an abnormality at the determination time in a case where the second determination feature amount does not satisfy second threshold value conditions.

In the medical information processing device according to the present invention, the determination section can classify the second determination feature amount into any one of a worsening trend, an improving trend, and other trends. The second determination section can determine whether or not the second determination conditions are satisfied in a case where the second determination feature amount is a worsening trend. The first determination section can determine whether or not the first determination conditions are satisfied in a case where the second determination feature amount is an improving trend. The first determination section can determine whether or not the additional first determination conditions are satisfied in a case where the second determination feature amount is other trends. The first determination conditions can be conditions in which the comparison feature amount is the determination time comparison feature amount, which is a value of an approximate curve corresponding to the determination time that is obtained based on the approximate curve calculated from a plurality of pieces of the inspection data corresponding to a fifth inspection item that is a predetermined inspection item in the first stable dosing period, and in which a difference between the determination time comparison feature amount and the first determination feature amount or a ratio between the determination time comparison feature amount and the first determination feature amount does not satisfy third threshold value conditions. The additional first determination conditions can be conditions in which the comparison feature amount is an average value of a plurality of pieces of the inspection data corresponding to a sixth inspection item that is a predetermined inspection item in the first stable dosing period and in which it is determined that there is an abnormality at the determination time in a case where a difference between the average value and the first determination feature amount or a ratio between the average value and the first determination feature amount does not satisfy fourth threshold value conditions.

In the medical information processing device according to the present invention, it is preferable to further include a second display control unit that displays the stable dosing period on a display screen, and it is preferable that the stable dosing period determination unit receives and acquires an input to change the displayed stable dosing period and changes the stable dosing period.

In the medical information processing device according to the present invention, it is preferable that the dosage details acquisition unit acquires drug specifying information in which each disease is associated with the disease associated drug, which is known to be administered for the disease, and selects and acquires the dosage details corresponding to the disease associated drug corresponding to the target disease based on the acquired drug specifying information.

In the medical information processing device according to the present invention, it is preferable that the determination section further includes a third determination section that determines whether or not third determination conditions for determining presence or absence of an abnormality at the determination time based on a difference between a value of the inspection data corresponding to a seventh inspection item that is a predetermined inspection item at the determination time and a value of inspection data corresponding to the seventh inspection item immediately before the determination time or a ratio between a value of the inspection data corresponding to an eighth inspection item that is a predetermined inspection item at the determination time and a value of inspection data corresponding to the eighth inspection item immediately before the determination time are satisfied. In the determination section, it is preferable that the third determination section determines whether or not the third determination conditions are satisfied and that the first determination section determines presence or absence of an abnormality at the determination time according to the first determination conditions only in a case where it is determined that there is no abnormality based on the third determination conditions.

In the medical information processing device according to the present invention, it is preferable that the determination section further includes a third determination section that determines whether or not third determination conditions for determining presence or absence of an abnormality at the determination time based on a difference between a value of the inspection data corresponding to a ninth inspection item that is a predetermined inspection item at the determination time and a value of inspection data corresponding to the ninth inspection item immediately before the determination time or a ratio between a value of the inspection data corresponding to a ninth inspection item that is a predetermined inspection item at the determination time and a value of inspection data corresponding to the ninth inspection item immediately before the determination time are satisfied. In the determination section, it is preferable that the third determination section determines whether or not the third determination conditions are satisfied, and the second determination section determines presence or absence of an abnormality at the determination time according to the second determination conditions only in a case where it is determined that there is no abnormality based on the third determination conditions.

The "target disease" may be acquired using an arbitrary method. For example, a disease extracted from the diagnostic information included in the medical information, such as the electronic medical record or the hospital discharge summary, can be used as a target disease. In addition, an input of the disease of the subject patient by the user may be received, and the received disease may be acquired as a target disease. In addition, a disease that is estimated from the past inspection information of the subject patient using an arbitrary technique may be used as a target disease.

The first to ninth inspection items described above may be the same inspection item or may be different inspection items as long as these are inspection items for which a desired feature amount can be calculated.

In the first to fourth threshold value conditions described above, the same type of feature amount may be used in the determination or different types of feature amount may be used in the determination as long as it is possible to determine a desired feature amount. In the first to fourth threshold value conditions described above, any value may be set as a threshold value as long as the value is a threshold value set so as to be able to perform desired determination based on the findings in the medical diagnosis. For example, in the first to fourth threshold value conditions, the same value may be set as a threshold value, or different values may be set as threshold values. In addition, in the first to fourth threshold value conditions, in order to determine the presence or absence of an abnormality, it may be determined that there is an abnormality in a case where the calculated value is equal to or greater than the threshold value or in a case where the calculated value is greater than the threshold value, and it may be determined that there is an abnormality in a case where the calculated value is equal to or less than the threshold value or in a case where the calculated value is less than the threshold value.

In the medical information processing device, method, and program of the present invention, a plurality of dosage details are acquired in which information specifying a drug administered to a subject patient, an amount of drug, and a dosing date of the drug are associated with each other. In addition, a dosing period equal to or longer than a predetermined period, among dosing periods of the drug for which it is regarded that both information specifying the drug and the amount of drug are the same, is identified based on the plurality of acquired dosage details, the identified dosing period is specified as a dosage details common period, and a period excluding a predetermined initial or final period from the specified dosage details common period is determined as a stable dosing period. Therefore, by using the stable dosing period as a period for which it can be estimated that treatment to maintain a stable state for a chronic disease is performed, it is possible to provide information useful for the treatment of the chronic disease by doctors. As a result, it is possible to improve the diagnostic accuracy and the diagnostic efficiency.

### BRIEF DESCRIPTION OF THE DRAWINGS

Fig. 1 is a diagram showing the schematic configuration of a medical information system using a medical information processing device according to a first embodiment of the present invention.
Fig. 2 is a block diagram showing the schematic configuration of the medical information processing device according to the first embodiment of the present invention.
Fig. 3 is a diagram showing a display example of the medical information display screen.
Fig. 4 is a diagram illustrating a method of determining a dosage details common period.
Fig. 5 is a diagram illustrating a method of determining a stable dosing period and a method of calculating a feature amount for first determination conditions.
Fig. 6 is a diagram illustrating a modification example of the method of calculating the feature amount for first determination conditions.
Fig. 7 is a diagram illustrating another modification example of the method of calculating the feature amount for first determination conditions.
Fig. 8 is a diagram illustrating a method of calculating a second determination feature amount.
Fig. 9 is a flowchart illustrating the flow of the process of the medical information system using the medical information processing device according to the first embodiment of the present invention.
Fig. 10 is a flowchart illustrating the flow of the process of a medical information system using a medical information processing device according to a second embodiment of the present invention.
Fig. 11 is a block diagram showing the schematic configuration of the medical information processing device according to a third embodiment of the present invention.
Fig. 12 is a flowchart illustrating the flow of the process of a medical information system using the medical information processing device according to the third embodiment of the present invention.

### DESCRIPTION OF THE PREFERRED EMBODIMENTS

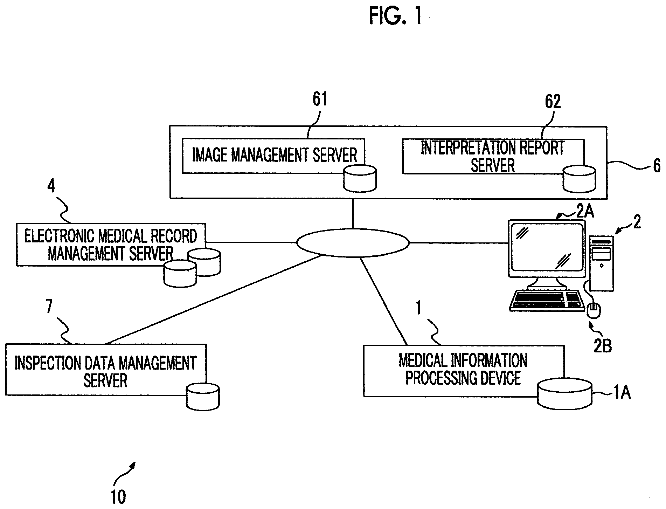

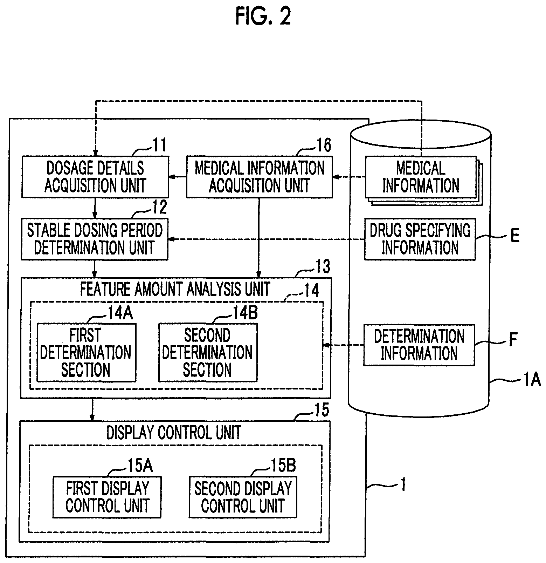

Hereinafter, an embodiment of a medical information processing device of the present invention will be described with reference to Figs. 1 and 2. Fig. 1 is a diagram showing the schematic configuration of a medical information system to which the medical information processing device according to the first embodiment of the present invention is applied, and Fig. 2 is a functional block diagram of the medical information processing device in one embodiment of the present invention.

As shown in Fig. 1, in a medical information system 10, a medical information processing device 1, a medical department terminal 2, an electronic medical record management server 4, a diagnostic imaging system 6, and an inspection data management server 7 are communicably connected to each other through a network.

The electronic medical record management server 4 is a computer including an electronic medical record database in which an electronic medical record is stored. In addition to the operating system or software for database management, software for searching for an image associated with each electronic medical record, medical information such as an inspection result, or the like and transmitting and receiving the search result in response to a request from the medical department terminal 2 or the like is installed in the electronic medical record management server 4. The electronic medical record management server 4 is connected to the medical information processing device 1, the medical department terminal 2, the inspection data management server 7, the diagnostic imaging system 6, and the like through a network so that the medical information managed through the electronic medical record can be acquired.

The inspection data management server 7 is a computer including an inspection data management database in which inspection data is stored. In addition to standard software, such as an operating system, software for managing inspection data is installed in the inspection data management server 7. The inspection date of subject inspection conducted in the inspection room in accordance with the inspection order input from each medical department terminal 2 and the subject inspection information of the inspection result are input to an inspection terminal (not shown) disposed in each inspection room so as to be associated with the inspection order or the patient ID, and is stored in the inspection data management database stores through a network.

The diagnostic imaging system 6 is a known computer system. Here, an image management server 61 including a diagnostic imaging medical workstation (not shown), a modality (not shown), such as a CT or an MRI, an image database in which image data obtained by imaging in the modality, such as a CT or an MRI, is stored and an interpretation report server 62 including an interpretation report database in which an interpretation report including the interpretation result of images obtained by imaging is stored are communicably connected to each other through a network. A variety of image analysis processing software is installed in the diagnostic imaging medical workstation, and is configured to be able to perform various kinds of image analysis processing according to the diagnostic purpose and the target.

The medical department terminal 2 is a computer that a doctor or the like of the department uses for the viewing of medical information of the patient, input of the inspection order, or the like, and includes a display unit 2A as a typical display and an input unit 2B configured to include a keyboard and a mouse. The medical department terminal 2 is also used to display and refer to the medical information, such as result data of inspection conducted in each department, or a created electronic medical record, and standard software, such as an operating system, and application software for displaying medical information, such as a created electronic medical record, are installed therein.

In the present embodiment, the medical department terminal 2 transmits various instructions and, if necessary, data corresponding to the various instructions to the medical information processing device 1, which will be described later, when the input unit 2B of the medical department terminal 2, such as a mouse or a keyboard, receives an operation of giving an instruction to start medical information display by users, such as doctors or an operation of designating (or inputting) required information, such as a patient ID, and various instructions for the medical information display and required information according to the various instructions are input thereto. The medical information processing device 1 receives the various instructions (and data corresponding thereto if necessary), and transmits information required for the medical information display of the present embodiment, such as display setting information defining the display settings and a plurality of pieces of medical information corresponding to the input patient ID, to the medical department terminal 2. Then, the medical department terminal 2 receives such display setting information and required information, and displays a medical information display screen, which will be described later, on the display screen of the display unit 2A of the medical department terminal 2 based on the received display setting information and required information.

The medical information processing device 1 is a computer including a medical information management database 1A. An operating system or software for database management is installed in the medical information processing device 1. Accordingly, the medical information processing device 1 also has a function as a management server of medical information. The medical information processing device 1 is connected to the electronic medical record management server 4, the medical department terminal 2, the inspection data management server 7, the image management server 61, and the interpretation report server 62 through a network, acquires medical information of a patient, such as an electronic medical record, various kinds of inspection result data, image data, and an interpretation report from each connected server by search based on the patient ID, and stores the acquired medical information in the medical information management database 1A so as to be associated with each patient ID. In each piece of medical information, each piece of medical data included in the medical information is stored so as to be associated with the inspection time (inspection date or the like). As medical information, it is possible to mention inspection information such as subject inspection information or image inspection information, treatment information such as dosage details, diagnostic information such as an electronic medical record or a hospital discharge summary in which information regarding the diagnosis, such as diagnosis of a display target person, symptoms, and surgical treatment, is recorded by doctors, biological information of a patient such as the body temperature, blood pressure, and respiration rate of the display target person, and image inspection information such as images captured by various modalities, such as an ultrasound diagnostic apparatus, a CT apparatus, an MRI apparatus, and a CR apparatus, and the interpretation reports. The medical information processing device 1 updates the medical information to be managed on time every day. In addition, the medical information processing device 1 receives and updates each piece of the medical information transmitted from each server or the like appropriately in response to a request from each server or the like, and acquires and updates each piece of the medical information from each server or the like appropriately depending on the need of the medical information processing device.

As shown in Fig. 2, a medical information display control program according to the present embodiment is installed in the medical information processing device 1 of the present embodiment. By the execution of the medical information display control program, the medical information processing device 1 functions as a dosage details acquisition unit 11, a stable dosing period determination unit 12, a medical information acquisition unit 16, a feature amount analysis unit 13, and a display control unit 15.

The medical information acquisition unit 16 acquires the patient ID transmitted from the medical department terminal 2, and acquires a plurality of pieces of medical information corresponding to the patient ID from the medical information management database 1A.

In addition, in each embodiment of this specification, the medical information acquisition unit 16 acquires all pieces of medical information associated with the patient ID from the medical information management database 1A based on the patient ID. Here, diagnostic information such as an electronic medical record, image inspection information, subject inspection information, biological information, and treatment information are acquired.

In the medical information management database 1A of the medical information processing device 1, display setting information required to display a plurality of pieces of medical information on the medical department terminal 2 in a predetermined display format is stored. In the medical information management database 1A, drug specifying information E obtained by selecting a drug action number corresponding to a drug used in the treatment of a disease on medical diagnosis and associating the drug action number with the disease by doctors is created for each disease and is stored. In addition, in the medical information management database 1A, determination information F, which is a composite table in which a disease, period specifying information, feature amount determination information, and determination conditions are associated with each other, is created in advance based on the findings in the medical diagnosis and is stored.

The dosage details acquisition unit 11 acquires information specifying the drug administered to the subject patient and a plurality of dosage details in which the amount of drug and the dosing date of the drug are associated with each other. Here, the dosage details acquisition unit 11 acquires a target disease that is the disease of the subject patient.

The "information specifying the drug" includes all pieces of information that can specify the type of drug. As an example, the information specifying the drug may be a therapeutic category number, or may be a drug code, or may be a drug name.

The "amount of drug" means the amount of drug administered to a patient in a predetermined unit of time. For example, it is preferable that the amount of drug shows the amount of drug on a daily basis. In the case of administering the drug multiple times in a day, the total amount of administration in a day can be set as the amount of drug. In the case of one administration in a week, the amount of drug administered by one administration may be averaged on a daily basis and the result may be set as the amount of drug, or the amount of drug on a weekly basis may be set as the amount of drug.

The "dosing date of the drug" means a day on which the drug is taken by being administered to the patient. For example, in the case of a drug administered by injection, the date of injection of the drug can be set as the dosing date of the drug. In the case of a prescribed drug, it is possible to specify the amount of drug to be taken per day and the taking date based on information or the like at the time of formulation and set the taking date as the dosing date of the drug.

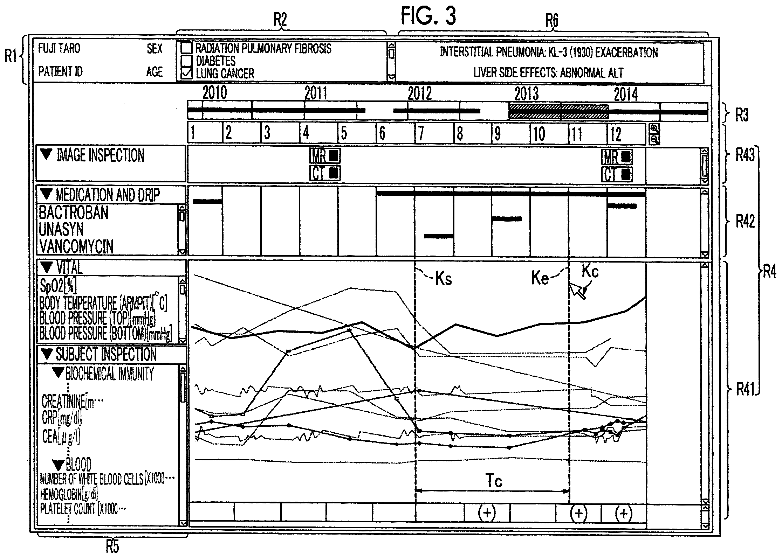

The dosage details acquisition unit 11 extracts a disease of the subject patient from the medical information, such as an electronic medical record of the subject patient or a hospital discharge summary, and instructs the display control unit 15 to display the extracted disease list of the patient. Although details will be described later, Fig. 3 shows an example in which a disease list is displayed in a disease list display field R2 on the medical information display screen where the medical information is displayed in time series. Then, the dosage details acquisition unit 11 receives an input of selecting a disease in the disease list from the user, and acquires the disease of the subject patient.

The "target disease" may be acquired using an arbitrary method. For example, a disease extracted from the diagnostic information included in the medical information, such as the electronic medical record or the hospital discharge summary, can be used as a target disease. In addition, an input of the disease of the subject patient by the user may be received, and the received disease may be acquired as a target disease. In addition, a disease that is estimated from the past inspection information of the subject patient using an arbitrary technique may be used as a target disease.

The dosage details acquisition unit 11 acquires the drug specifying information E in which a disease associated drug, which is known to be administered for a disease, is associated with each disease, specifies a disease associated drug corresponding to the acquired target disease based on the acquired drug specifying information E, and selects and acquires the dosage details corresponding to the specified disease associated drug. Thus, in a case where the dosage details acquisition unit 11 acquires a disease of the subject patient and acquires selectively the dosage details for a drug known to be used for the treatment since there is an effect on the disease, a period for which the dosage details for the treatment of chronic diseases is common can be appropriately extracted even in a case where the dosage details for other diseases is present for the subject patient.

Table 1 shows an example of the drug specifying information E. The drug specifying information E in Table 1 is created by associating a therapeutic category number with a disease by user setting. In general, drugs are classified according to the effect of each drug, and a therapeutic category number corresponding to the classification of the effect of the drug is given to each drug. In Table 1, a drug action corresponding to each therapeutic category number is shown in a supplementary field for reference. The dosage details acquisition unit 11 specifies a therapeutic category number corresponding to the target disease of the subject patient based on the drug specifying information E, specifies a drug corresponding to the specified therapeutic category number as a disease associated drug, and specifies a dosage details common period based on the specified disease associated drug. Thus, in the case of using the drug specifying information E in which information specifying a drug action is associated with diseases, all disease associated drugs corresponding to the information specifying a drug action can be associated with diseases. Therefore, since it is possible to prevent the omission of detection of a disease associated drug, it is possible to accurately specify a disease associated drug corresponding to each disease.

**[Table 1]**

| Disease | Therapeutic category number | Supplement (drug action) |
|---|---|---|
| Interstitial pneumonia | 245 | Adrenal hormone preparations |
| | 611 | Antibiotic formulation that acts mainly on gram-positive bacteria |
| | 612 | Antibiotic formulation that acts mainly on gram-negative bacteria |
| | 613 | Antibiotic formulation that acts mainly on gram-positive and gram-negative bacteria |
| | 629 | Other chemotherapeutic agents |
| | ... | ... |
| | 421 | Alkylating agent |
| | 422 | Metabolic antagonist |
| | 423 | Antitumor antibiotic formulation |
| | ... | ... |
| Bacterial pneumonia | 245 | Adrenal hormone preparations |
| | 611 | Antibiotic formulation that acts mainly on gram-positive bacteria |
| | ... | ... |
| ... | ... | ... |

Table 2 shows an example of a table of correspondence between the therapeutic category number and the drug code, and Table 3 shows an example of drugs included in a plurality of dosage details of the subject patient. Here, each therapeutic category number corresponding to the interstitial pneumonia that is a target disease of the subject patient is specified based on the drug specifying information E in Table 1, a drug code corresponding to the specified therapeutic category number is specified based on the correspondence table in Table 2, and Bactroban, Vancomycin, and Unasyn corresponding to the drug code are specified as disease associated drugs. In addition, as shown in Table 3, disease associated drugs included in the dosage details of the subject patient are specified.

**[Table 2]**

| Drug name | Drug code | Therapeutic category number |
|---|---|---|
| Bactroban | 6290100F2115 | 629 |
| Vancomycin | 6113001B1097 | 611 |
| Unasyn | 6131008F1030 | 613 |
| PL | 1180107D1131 | 118 |
| Loxonin | 1149019F1560 | 114 |

**[Table 3]**

| Name of drug administered to subject patient | Drug code | Equivalent to disease associated drug? |
|---|---|---|
| Bactroban | 6290100F2115 | Yes |
| Vancomycin | 5113001B1097 | Yes |
| Unasyn | 6131008F1030 | Yes |
| PL | 1180107D1131 | No |
| Loxonin | 1149019F1560 | No |

In the drug specifying information E, if a drug (or information capable of specifying the drug, such as a therapeutic category number) known to be used for the treatment since there is an effect on the disease is associated with the disease, the disease and the drug (or information capable of specifying the drug, such as a therapeutic category number) may be associated with each other using an arbitrary method. For example, for each subject patient, drugs that the user thinks to be applied for the treatment of chronic diseases may be selected and set. In this case, there is a setting burden on the user. However, since user determination is performed, it is possible to appropriately specify drugs used for the treatment of chronic diseases and appropriately extract a stable dosing period, for which the dosage details is common, for specific drugs for maintaining the chronic diseases stably. In addition, the drug specifying information E may be generated by automatically extracting a disease and the dosage details corresponding to the disease using an arbitrary method after analyzing a plurality of pieces of past medical information of the patient. For a target disease or a disease in the drug specifying information E, for example, a disease name, a symptom name, or a syndrome name can be used. Alternatively, a disease name classified into a detailed small category may be used, or a disease name in a middle category or a large category that is a higher-level category may be used.

The stable dosing period determination unit 12 identifies a dosing period equal to or longer than a predetermined period, among dosing periods of the drug for which it can be regarded that both the information specifying the drug and the amount of drug are the same, based on the plurality of acquired dosage details, specifies the identified dosing period as a dosage details common period, and determines a period excluding a predetermined initial or final period from the specified dosage details common period as a stable dosing period.

The "dosing period of the drug for which it can be regarded that both the information specifying the drug and the amount of drug are the same" is preferably a period for which it can be regarded that both the information specifying the drug and the dose of the drug are substantially the same. In a case where treatment to maintain a stable state for a chronic disease is performed on the patient, a specific drug is applied to the patient over a period equal to or longer than a predetermined period while maintaining a fixed amount of drug in many cases. Therefore, by specifying a period for which both the information specifying the drug and the dose of the drug are the same, it is possible to specify the implementation period of treatment to maintain a stable state for a chronic disease. Here, in a case where it can be regarded that the information specifying substantially the same drug and the dose of the drug are maintained and managed for the patient by the doctor, it is regarded that both the information specifying the drug and the dose of the drug are the same. For example, for a case where a period for which the patient forgets to take a prescribed medicine temporarily occurs or a case where an interval at which the patient is subjected to medical examination extends and taking the prescribed medicine ends and accordingly a no-dosing period temporarily occurs, it can be regarded that both the information specifying the drug and the dose of the drug are substantially the same. In addition, there may be a case where doctors finely adjust the amount of drug while observing the condition of the patient. Accordingly, for a change in the amount of drug equal to or less than a predetermined threshold value, it can be regarded that that both the information specifying the drug and the dose of the drug are substantially the same. In addition, also for a case where the original drug is replaced with generic drug, it can be regarded that that both the information specifying the drug and the dose of the drug are substantially the same. The information specifying substantially the same drug may indicate a common drug action (therapeutic category number).

For the "dosing period equal to or longer than a predetermined period among dosing periods of the drug for which it can be regarded that both the information specifying the drug and the amount of drug are the same", a period for which it can be estimated that treatment to maintain a stable state for a chronic disease is performed can be set as the predetermined period. For example, the predetermined period can be set to several weeks to several months or more.

The "stable dosing period" means a period excluding at least one of a predetermined initial period and a predetermined final period from the dosage details common period. In the dosage details common period for maintaining a stable state for a chronic disease, in the predetermined initial period, there is a possibility that changes in the symptoms due to a change in the treatment details from the treatment before medication will occur. In addition, in the predetermined final period, there is a possibility that changes in the symptoms will occur, for example, since the dosage details common period ends due to a change in the dosage details in the dosage details common period. Therefore, by setting the period excluding at least one of the predetermined initial period and the predetermined final period as a stable dosing period, it is possible to appropriately use the stable dosing period as a period for checking the stable state of the chronic disease. In addition, as the stable dosing period, it is preferable that a period, for which it is possible to extract the features of inspection data, based on the findings in the medical diagnosis. For example, it is preferable to set an appropriate period as a stable dosing period for each disease or each determination purpose.

In addition, the "predetermined initial period" is a period excluding a period in which it is considered that changes in the symptoms due to a change in the treatment details from the treatment before medication occur, and may be a period of one or more days set in advance. It is preferable that the "predetermined initial period" is appropriately set in accordance with the details required for the disease of the patient or the dosage details common period. In addition, the "predetermined final period" is a period excluding a period in which it is considered that changes in the symptoms occur, for example, since the dosage details common period ends due to a change in the dosage details in the dosage details common period, and may be a period of one or more days set in advance. It is preferable that the "predetermined final period" is appropriately set in accordance with the details required for the disease of the patient or the dosage details common period.

The stable dosing period determination unit 12 acquires a determination time Tt and the determination purpose. Here, on the display screen shown in Fig. 3 that shows the medical information of the display unit 2A of the medical department terminal 2 in time series, the user designates a desired time on the time line of an inspection period display field R3 to display the presence or absence of inspection information of the subject patient along the time axis, and performs an input to determine the designated time as the determination time Tt. Then, the medical department terminal 2 receives a time designated as the determination time Tt and transmits the time to the medical information processing device 1, and the stable dosing period determination unit 12 of the medical information processing device 1 receives the time and acquires the designated time as the determination time Tt.

By the display control unit 15, a determination purpose list (not shown) for the selection of the determination purpose is dialog-displayed on the display screen of the display unit 2A of the medical department terminal 2. Then, the medical department terminal 2 receives a selection of the determination purpose by the user and transmits this to the medical information processing device 1, and the stable dosing period determination unit 12 of the medical information processing device 1 receives this to acquire the selected determination purpose.

The "determination time Tt" may be acquired using an arbitrary method. For example, a medical examination date may be acquired as the determination time Tt, or a numerical input of the determination time Tt by the user may be received on the display screen, and the received time may be acquired as the determination time Tt. In addition, the "determination purpose" may be acquired using an arbitrary method. For example, for the determination purpose, a text input of the determination purpose by the user may be received on the display screen, and the received text information may be acquired as the determination purpose.

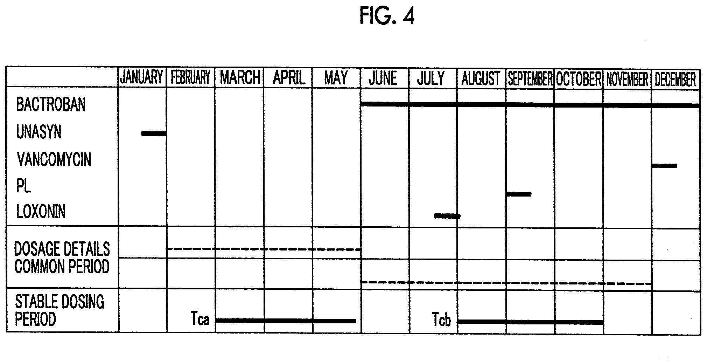

Fig. 4 is a diagram showing the dosage details of a subject patient in time series. Here, the stable dosing period determination unit 12 extracts a dosing period equal to or longer than a predetermined period, among dosing periods of the drug for which it can be regarded that both the information specifying the drug and the amount of drug are the same, for Bactroban, Vancomycin, and Unasyn specified as disease associated drugs in the example shown in Table 2. In the example shown in Fig. 4, a period from February to May, in which none of Bactroban, Vancomycin, and Unasyn have been administered, and a period from June to November, in which Bactroban has been administered and Vancomycin and Unasyn have not been administered, are extracted as dosage details common periods. As shown in Fig. 4, as an example, the stable dosing period determination unit 12 can determine a period excluding a predetermined initial period (one month) and a predetermined final period (one week) from the dosage details common period as a stable dosing period Tca.

Incidentally, for diseases with similar symptoms that may be affected together, such as interstitial pneumonia and bacterial pneumonia, there are cases in which drugs used in the treatment are the same. For example, as shown in Table 2, steroid and antibiotic preparations may be administered for both diseases of bacterial pneumonia and interstitial pneumonia. For this reason, the administration of Vancomycin, which is an antibiotic preparation, in the first half of December shown in Fig. 4 may be administration due to a change in the symptoms of interstitial pneumonia, and may also be administration due to the symptoms of bacterial pneumonia temporarily affected. Therefore, if a dosage details common period is determined based on the disease associated drug corresponding to interstitial pneumonia, the dosage details common period is determined so as to exclude a period for which a change in the symptoms of bacterial pneumonia, which is a disease for which drugs in common with interstitial pneumonia are used, occurs.

Preferably, in a case where it is necessary to eliminate a change in symptoms that has occurred immediately before the determination time Tt, the stable dosing period determination unit 12 determines a first stable dosing period, which is a stable dosing period before the determination time Tt, so as to be able to eliminate the change in the symptoms that has occurred immediately before the determination time Tt. In this case, since a possibility that the change in the symptoms of the subject patient, which has occurred immediately before the determination time Tt, has been eliminated is high, a possibility that the first stable dosing period will be a period, for which the symptoms of the chronic disease are stable, is high. For this reason, by using the inspection data included in the first stable dosing period, it is possible to appropriately extract the features of inspection data in a state in which the symptoms of the subject patient are stable. Therefore, by calculating the feature amount indicating the trend of the inspection data of a predetermined inspection item in the first stable dosing period and determining the difference between the calculated feature amount and inspection data of a predetermined inspection item at the determination time, it is possible to appropriately determine the features of the inspection data when the symptoms of the chronic disease of the subject patient are stable and the features of the inspection data at the determination time.

In a case where a first stable dosing period is calculated for each of a plurality of common dosing periods, it is preferable that the stable dosing period determination unit 12 acquires the determination time Tt using an arbitrary method and determines a stable dosing period immediately before the determination time Tt, among the plurality of common dosing periods, as a first stable dosing period Tc for use in the abnormality determination of the determination time Tt. In the case of inspection data of a period too far from the determination time Tt, a possibility that there is a change in the condition of the subject patient for various reasons is high. Therefore, by excluding the inspection data in a period too far from the determination time Tt from feature amount calculation targets, setting a period close enough to be acceptable from the determination time Tt as the first stable dosing period Tc, and determining the presence or absence of an abnormality of the determination time Tt based on the inspection data included in the period, the abnormality of the subject patient of the determination time Tt can be determined more accurately.

In order to further enhance the above effect, it is preferable that the stable dosing period determination unit 12 further determines a period having a start time after a first reference time T1 that defines a predetermined period before the determination time Tt and having an end time before a second reference time T2 that is after the start time and before the determination time Tt, among the first stable dosing periods immediately before the determination time, as a comparison stable dosing period Tc.

As the first reference time T1, it is preferable to set a time determined that it is possible to limit a time close enough to be acceptable from the determination time Tt, excluding the inspection data in a period too far from the determination time Tt from feature amount calculation targets. In a case where it is necessary to eliminate a change in symptoms that has occurred immediately before the determination time Tt, it is preferable that a time determined that it is possible to eliminate the change in symptoms that has occurred immediately before the determination time Tt is set as the second reference time T2. It is preferable that an appropriate time is set as each of the first reference time T1 and the second reference time T2, for example, for each disease (or each combination of a disease and a determination purpose). By setting the first reference time and the second reference time appropriately according to the disease or the desired determination purpose, it is possible to appropriately determine a comparison stable dosing period.

On the other hand, in a case where it is necessary to observe a change in symptoms that has occurred immediately before the determination time Tt, it is preferable that the stable dosing period determination unit 12 determines a second stable dosing period that is a stable dosing period including the determination time Tt in the final stage. Thus, in the second stable dosing period that is a stable dosing period set to include the determination time in the end stage, the change in the symptoms of the subject patient that has occurred immediately before the determination time is considered to be reflected in the inspection data. Therefore, a possibility that the change in symptoms from the start of the second stable dosing period to the determination time can be checked by calculating the feature amount indicating the trend of the inspection data of a predetermined inspection item in the second stable dosing period and determining the calculated feature amount is high.

In addition, the stable dosing period determination unit 12 can determine a period, which has a start time after the additional first reference time that defines a predetermined period before the determination time Tt and has an end time before the additional second reference time after the determination time Tt, as a second stable dosing period.

Similar to the first reference time T1, as the additional first reference time T1, it is preferable to set a time determined that it is possible to limit a time close enough to be acceptable from the determination time Tt, excluding the inspection data in a period too far from the determination time Tt from feature amount calculation targets. As the additional second reference time T2, in order to check the change in symptoms that has occurred immediately before the determination time Tt, it is preferable that a time including the change in symptoms that has occurred immediately before the determination time Tt is appropriately set. For example, the second reference time T2 may be the same day as the determination time Tt, or the second reference time T2 may be one day to one week after the determination time Tt. Alternatively, a predetermined period of the second stable dosing period, such as the last 1% of the entire period, may be set as a final stage, and the second reference time may be determined so as to include the determination time Tt in the final stage. It is preferable that an appropriate time is set as each of the additional first reference time T1 and the additional second reference time T2 from the end, for example, for each disease (or each combination of a disease and a determination purpose). By setting the additional first reference time and the additional second reference time appropriately according to the disease or the desired determination purpose, it is possible to appropriately determine the second stable dosing period.

As an example, in Fig. 4, assuming that the determination time Tt is December 1, June to November are a dosage details common period, and July to October excluding the initial one month and the last one month, which are predetermined periods, from the period of June to November can be determined as a first stable dosing period immediately before the determination time Tt. In addition, assuming that the first reference time is four months and the second reference time is one month, a period excluding the final one month, which is a period less than four months from the determination time Tt, from the stable dosing period immediately before the determination time Tt can be determined as a comparison stable dosing period Tcb.

Here, the stable dosing period determination unit 12 acquires a composite table that is the determination information F in which a disease, determination purpose, period specifying information, feature amount specifying information to be described later, and determination conditions to be described later are associated with each other, specifies the period specifying information corresponding to the target disease and the determination purpose based on the composite table, and determines a stable dosing period (comparison stable dosing period Tc or second stable dosing period) according to the specified period specifying information. Thus, since the stable dosing period determination unit 12 specifies the period specifying information corresponding to the target disease and the determination purpose based on the composite table in which the target disease, the determination purpose, and the period specifying information are associated with each other and determines the stable dosing period according to the specified period specifying information, it is possible to determine the appropriate stable dosing period corresponding to the target disease and the determination purpose. Instead of the correspondence table, a correspondence table in which the target disease, the determination purpose, and the period specifying information are associated with each other may be used. Here, the period specifying information is configured to include the first reference time and the second reference time.

Table 4 shows an example of the composite table. The composite table may be configured as a plurality of tables instead of one table if it is possible to individually refer to association information in which a disease, a determination purpose, and period specifying information are associated with each other, association information in which a disease, a determination purpose, and feature amount specifying information are associated with each other, and association information in which a disease, a determination purpose, and determination conditions are associated with each other. In the period specifying information, additional information may be associated in addition to the disease and the determination purpose, and association with the determination purpose may be omitted. Similarly, in the feature amount specifying information, additional information may be associated in addition to the disease and the determination purpose, and association with the determination purpose may be omitted. In addition, in the determination conditions, additional information may be associated in addition to the disease and the determination purpose, and association with the determination purpose may be omitted.

In Table 4, a first reference time and an additional first reference time are written without distinction, and a second reference time and an additional second reference time are written without distinction. In Table 4, a case where the second reference time T2 is later than the determination time Tt is an example in which the first stable dosing period, which is a stable dosing period before the determination time Tt, is determined, and a case where the determination time Tt is the same or later than the second reference time T2 is an example in which the second stable dosing period, which is a stable dosing period including the determination time Tt, is determined. In order to make the determination time Tt later than the second reference time T2, the second reference time is set to minus in Table 4.

The period specifying information may define a predetermined period using an arbitrary method as long as it is possible to specify a predetermined period. For example, a stable dosing period may be defined by the first reference time (or the second reference time) and a predetermined period. In addition, the feature amount specifying information may define the feature amount using an arbitrary method as long as it is possible to specify a method of calculating the feature amount from the inspection item and inspection data. In addition, the determination conditions may be any conditions under which abnormalities can be determined based on the feature amount.

**[Table 4]**

| Disease | Determination purpose | First reference time | Second reference time | Inspection item | Feature amount conditions (first determination feature amount, comparison feature amount, or second determination feature amount) | Determination method | Threshold value |
|---|---|---|---|---|---|---|---|
| interstitial pneumonia | acute exacerbation | 6 | 1 | KL-6 (sialylated carbohydrate antigen KL-6) | Inspection data at determination time, average value of specific inspection data | Ratio of first determination feature amount to comparison feature amount is equal to or less. than threshold value | 1.35 |
| | | 6 | 1 | SP-D (pulmonary surfactant apoprotein-D) | Inspection data at determination time, average value of specific inspection data | Ratio of first determination feature amount to comparison feature amount is equal to or less than threshold value | 1.5 |
| | | 6 | 1 | SP-A (pulmonary surfactant apoprotein-A) | Inspection data at determination time, average value of specific inspection data | Ratio of first determination feature amount to comparison feature amount is equal to or less than threshold value | 1.35 |
| | chronic exacerbation | 6 | 0 | KL-6 | Rate of change in specific inspection data | Second determination feature amount is equal to or less than threshold value | 1.1 /month |
| | | 6 | 0 | SP-D | Rate of change in specific inspection data | Second determination feature amount is equal to or less than threshold value | 1.2/month |
| | | 6 | 0 | SP-A | Rate of change in specific inspection data | determination feature amount is equal to or less than threshold value | 1.1/month |
| | Side effects | 6 | 1 | AST aminotransferase) | Inspection data at determination time, value of specific inspection data | Difference between comparison feature amount and first determination feature amount is equal to or less than threshold value | 50UI/1 |
| | ... | ... | ... | ... | | ... | ... |
| Lung cancer | Exacerbation | 10 | 0 | CEA (carcinoembryonic antigen) | Rate of change in specific inspection data | Second determination feature amount is equal to or less than threshold value | 1.4 |
| | | 10 | 0 | SCC antigen (squamous cell carcinoma associated antigen) | Rate of change in specific inspection data | Second determination feature amount is equal to or less than threshold value | 1.4 |
| | Side effects | 6 | 1 | AST | Inspection data at determination time, average value of specific inspection data | Difference between comparison feature amount and first determination feature amount equal to or less than threshold value | 50UI/1 |
| | ... | ... | ... | ... | | ... | ... |

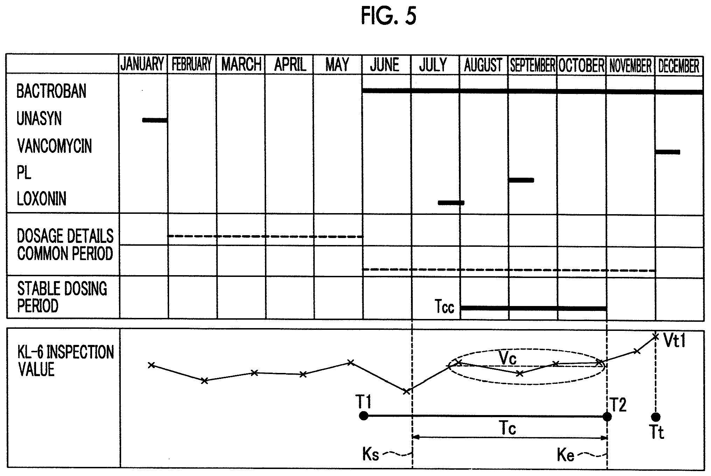

Using Table 4 and Fig. 5, a determination method for determining a stable dosing period based on the first reference time (or the additional first reference time) and the second reference time (or the additional second reference time) that are acquired using the composite table will be described. In the example shown in Fig. 5, it is assumed that the determination time is December 1, and the disease of the subject patient is interstitial pneumonia, and acute exacerbation is selected as the determination purpose. Then, based on the period specifying information shown in Table 4, the first reference time T1 corresponding to interstitial pneumonia and acute exacerbation is six months, and the second reference time T2 is one month. That is, as shown in Fig. 5, a first stable dosing period from July to November excluding one month, which is a predetermined initial period, from the dosage details common period from June to November and a first stable dosing period from March to May of the dosage details common period from February to May are specified. In addition, between these first stable dosing periods, a first stable dosing period Tcc immediately before the determination time Tt is specified. Of the first stable dosing period Tcc immediately before the determination time Tt, a period from July to October, which is a period after six months from the determination time Tt and before one month from the determination time Tt, is specified as a comparison stable dosing period Tc. In addition, although not shown in Fig. 5, a method of determining the second stable dosing period using Table 4 will be described. For example, assuming that the determination time is December 1, the disease of the subject patient is interstitial pneumonia, and chronic exacerbation is selected as the determination purpose, the first reference time T1 and the second reference time T2 corresponding to the interstitial pneumonia and the chronic exacerbation are six months and zero month, respectively, based on the period specifying information shown in Table 4. In addition, a period that starts before six months from the determination time Tt and ends at the determination time Tt is specified as the second stable dosing period.

The stable dosing period determination unit 12 receives and acquires an input for changing a stable dosing period displayed by the display control unit 15 to be described later, and updates the stable dosing period to the changed period and determines the changed period as a new stable dosing period in a case where there is a change in the stable dosing period. This function will be described in detail in the explanation of a second display control section 15B.

The feature amount analysis unit 13 extracts medical information corresponding to the inspection time included in the stable dosing period, of the acquired medical information, as specific medical information, and acquires the feature amount from specific inspection data that is inspection data corresponding to the extracted specific medical information.

In addition, the feature amount analysis unit 13 includes a determination section 14 that determines the presence or absence of an abnormality of the subject patient using the feature amount. The determination section 14 includes: a first determination section 14A that acquires a comparison feature amount Vc determined from the specific inspection data included in the first stable dosing period, which is a stable dosing period before the determination time Tt of the subject patient, and a first determination feature amount Vt1 determined from the inspection data at the determination time of the subject patient and that determines the presence or absence of an abnormality of the subject patient based on the acquired comparison feature amount and the acquired first determination feature amount; and a second determination section 14B that determines the presence or absence of an abnormality of the subject patient based on a second determination feature amount Vt2 determined from a plurality of pieces of inspection data of the subject patient included in the second stable dosing period that is a stable dosing period including the determination time Tt of the subject patient in the final stage.

Based on the composite table shown in Table 4, the feature amount analysis unit 13 acquires feature amount specifying information corresponding to the disease of the subject patient, the determination purpose, and the determination time Tt. Here, the feature amount information is configured to include an inspection item and a method of specifying the features amount from inspection data corresponding to the inspection item. Then, the feature amount analysis unit 13 acquires the inspection data of a predetermined inspection item included in the stable dosing period, as specific inspection data, based on the feature amount specifying information, and acquires the feature amount from the specific inspection data.

As the feature amount, any feature amount may be used as long as the feature amount can be extracted from the inspection data of the stable dosing period and the presence or absence of an abnormality at the determination time can be determined.

In addition, as inspection items used for the calculation of each feature amount, one or more inspection items of medical treatment items included in the target medical information may be arbitrarily set as long as it is possible to calculate a desired feature amount.

In a case where the stable dosing period determined by the stable dosing period determination unit 12 is a first stable dosing period including no determination reference time, the first determination section 14A acquires the comparison feature amount of the first stable dosing period, which is the feature amount of the first stable dosing period, from the inspection data corresponding to a predetermined inspection item included in the first stable dosing period Tc, and acquires a first determination target feature amount based on the determination time Tt. The first determination section 14A may acquire the comparison feature amount based on only the inspection data corresponding to a predetermined inspection item included in the first stable dosing period immediately before the determination time of the first stable dosing period Tc. In addition, the first determination section 14A may acquire the comparison feature amount based on only the inspection data corresponding to a predetermined inspection item included in the comparison stable dosing period of the first stable dosing period immediately before the determination time.

Hereinafter, an example of acquiring the comparison feature amount and the first determination feature amount based on only the inspection data corresponding to a predetermined inspection item included in the comparison stable dosing period will be described. For example, in Table 4, it is assumed that an inspection item KL-6 is specified as feature amount specifying information corresponding to interstitial pneumonia that is a target disease and acute exacerbation that is a determination purpose, the first determination feature amount is inspection data at the determination time, and the comparison feature amount is specified as an average value of specific inspection data. Table 5 shows an example of the inspection data of the inspection item KL-6 of the subject patient.

In the above case, as shown by a bold frame in Table 5, inspection data for 4 times whose inspection dates are in July to October is extracted as specific inspection data. Such specific inspection data corresponds to inspection data indicated by the ellipse in Fig. 5. The feature amount analysis unit 13 acquires the average value of the four pieces of specific inspection data as the comparison feature amount Vc (= 1332.5), and acquires the inspection data of the determination time Tt shown in a double frame portion of Table 5 as the first determination feature amount Vt1 (= 1930). Similarly, as shown in Table 5, also for inspection items SP-D and SP-A associated with interstitial pneumonia and acute exacerbation, the feature amount analysis unit 13 acquires the first determination feature amount and the comparison feature amount based on the feature amount specifying information defined in Table 4.

**[Table 5]**

| Inspection date | Inspection data | Supplement |
|---|---|---|
| **12/1** | **1930** | **Inspection data at determination target time** |
| 11/15 | 1690 | Since it is inspection date after second reference time, inspection data that is not included in comparison stable dosing period |
| **10/15** | **1380** | **Inspection data in comparison stable dosing period** |
| **9/15** | **1340** | **Inspection data in comparison stable dosing period** |
| **8/15** | **1220** | **Inspection data in comparison stable dosing period** |
| **7/15** | **1390** | **Inspection data in comparison stable dosing period** |
| 6/15 | 1050 | Since it is inspection date that is after first reference time but is not included in common dosing period, inspection data that is not included in comparison stable dosing period |
| 5/15 | 1380 | Since it is inspection date before first reference time, inspection data that is not included in comparison stable dosing period |
| 4/15 | 1260 | Since it is inspection date before first reference time, inspection data that is not included in comparison stable dosing period |
| 3/15 | 1270 | Since it is inspection date before first reference time, inspection data that is not included in comparison stable dosing period |
| 2/15 | 1190 | Since it is inspection date before first reference time, inspection data that is not included in comparison stable dosing period |
| 1/15 | 1300 | Since it is inspection date before first reference time, inspection data that is not included in comparison stable dosing period |

In a case where the feature amount analysis unit 13 acquires a target disease, the comparison feature amount Vc, and first determination feature amount Vt1, the first determination section 14A acquires the composite table shown in Table 4, and acquires determination conditions corresponding to the disease of the subject patient and the determination purpose based on the composite table. Then, in a case where the determination conditions are first determination conditions for determining the presence or absence of an abnormality of the determination time Tt based on the comparison feature amount and the first determination feature amount, the first determination section 14A determines whether or not the first determination conditions are satisfied based on the comparison feature amount and the first determination feature amount. In Table 4, the determination conditions include a determination method and a threshold value.

For example, in a case where the first determination feature amount Vt1 is set as the inspection data of the inspection item KL-6 at the determination time and the comparison feature amount Vc is set as the average value of the specific inspection data of the inspection item KL-6, the first determination section 14A acquires conditions that the ratio of the first determination feature amount Vt1 to the comparison feature amount Vc (Vt1/Vc) is equal to or less than the threshold value 1.35, as determination conditions corresponding to the interstitial pneumonia and the acute exacerbation described above, based on the composite table. Then, the first determination section 14A acquires the comparison feature amount Vc (= 1332.5) and the first determination feature amount Vt1 (= 1930), and determines whether or not the conditions of Vt1/Vc ≤ 1.35 are satisfied. In this example, since Vt1/Vc = 1.45 > 1.35, the first determination conditions are not satisfied. Accordingly, it is determined that there is an abnormality. Similarly, as shown in Table 4, also for the inspection items SP-D and SP-A associated with interstitial pneumonia and acute exacerbation, the first determination section 14A determines whether or not the determination conditions defined in Table 4 are satisfied. In addition, as shown in Table 4, depending on the inspection item, as a determination parameter, a difference between the comparison feature amount and the first determination feature amount may be used as an index value instead of the ratio between the comparison feature amount and the first determination feature amount.

As described above, in a case where the comparison feature amount is set as the average value of a plurality of pieces of specific inspection data in the first stable dosing period corresponding to a predetermined inspection item, the feature of inspection data included in a period of the state in which the symptoms of the chronic disease of the subject patient are relatively calm can be appropriately expressed by the comparison feature amount. Therefore, based on the first determination feature amount and the comparison feature amount, it is possible to appropriately determine a change at the determination time with respect to the first stable dosing period for the symptoms of the chronic disease.

As a method of calculating the comparison feature amount, it is possible to adopt various feature amount calculation methods according to the inspection item as long as it is possible to show the feature of specific inspection data of a desired inspection item. In addition, as long as it is possible to determine the abnormality at the time of determination, a comparison feature amount for any inspection item may be used. In addition, as long as the first determination feature amount can be specified from the predetermined inspection data of the determination time Tt and is a feature amount corresponding to the comparison feature amount, it is possible to apply any feature amount calculation method. In addition, the first determination feature amount may be a feature amount for any inspection item as long as the first determination feature amount corresponds to the comparison feature amount.

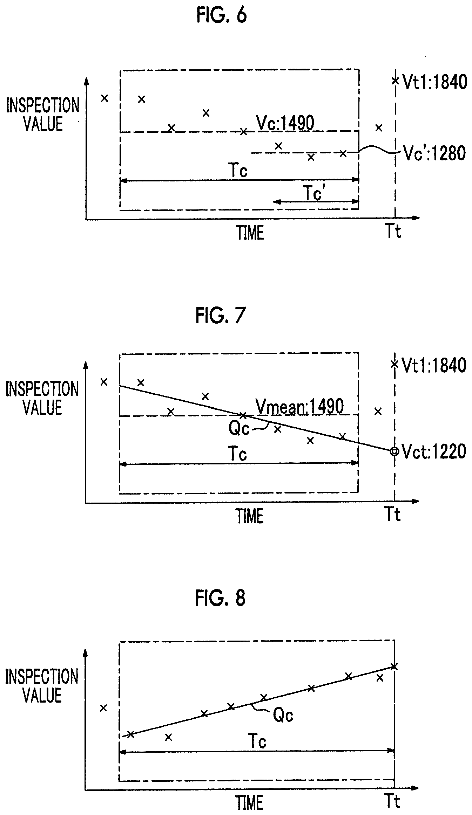

Figs. 6 and 7 show other examples of the method of calculating the comparison feature amount. For example, the feature amount analysis unit 13 may acquire the comparison feature amount Vc of the first determination conditions using a predetermined number of pieces of specific inspection data going back into the past in time series from the end of the comparison stable dosing period Tc. As shown in Fig. 6, it is possible to calculate the comparison feature amount Vc using three pieces of specific inspection data going back into the past in time series from the end of the comparison stable dosing period Tc.

In the case of calculating the comparison feature amount using a predetermined plurality of specific inspection data going back into the past in time series from the end of the comparison stable dosing period, in a case where the number of pieces of specific inspection data is large, it is possible to calculate the comparison feature amount by using specific inspection data having a determination time close to the inspection time preferentially. Therefore, by using this comparison feature amount, it is possible to perform determination of the subject patient more appropriately. In addition, by calculating the comparison feature amount using a predetermined plurality of specific inspection data, it is possible to reduce the influence of a variation in inspection data. According to this method, at the time of a stable state in which the specific inspection data of a period close to the determination time indicates the same value, the difference between the comparison feature amount and the first determination feature amount becomes noticeable. Therefore, it is possible to appropriately determine the abnormality of the subject patient.

As shown in Fig. 6, in a case where the average value of specific inspection data included in the comparison stable dosing period Tc is set as the comparison feature amount Vc of the first determination conditions, Vc = 1490, and first determination feature amount Vt1 = 1840. In addition, in a case where the average value of three pieces of specific inspection data going back into the past in time series from the end of the comparison stable dosing period Tc is set as a comparison feature amount Vc' of the first determination conditions, Vc' = 1280, and first determination feature amount Vt1 = 1840.

In a case where the first determination conditions are, for example, determination conditions defining that the absolute value of the difference (Vt1 - Vc) between Vc and Vt1 or the absolute value of the ratio (Vt1/Vc) of Vt1 to Vc is equal to or less than a predetermined threshold value condition, in the example shown in Fig. 6, the difference between the comparison feature amount and the first determination feature amount Vt1 in a case where the average value of three pieces of specific inspection data going back into the past in time series from the end of the comparison stable dosing period Tc is the comparison feature amount Vc' of the first determination conditions is more noticeable than that in a case where the average value of specific inspection data included in the comparison stable dosing period Tc is the comparison feature amount Vc of the first determination conditions. Accordingly, it is possible to appropriately determine the abnormality of the first determination feature amount Vt1. In the example shown in Fig. 6, instead of determining the comparison feature amount based on a predetermined number of pieces of specific inspection data going back into the past in time series from the end of the comparison stable dosing period, the comparison feature amount may be determined based on a predetermined number of pieces of specific inspection data going back into the past in time series from the end of the first stable dosing period immediately before the determination time.

As in the example shown in Fig. 7, the first determination conditions may be defined such that it is determined, based on an approximate curve Qc calculated from a plurality of pieces of specific inspection data in the first stable dosing period (for example, in the comparison stable dosing period Tc) corresponding to a predetermined inspection item, that there is an abnormality in a case where the difference between the first determination feature amount Vt and a determination time comparison feature amount Vct, which is a value of the approximate curve corresponding to the determination time Tt, or the ratio between the determination time comparison feature amount Vct and the first determination feature amount Vt does not satisfy predetermined threshold value conditions.

In a case where the comparison feature amount is a value of the approximate curve corresponding to the determination time in the approximate curve calculated from a plurality of pieces of specific inspection data in the first stable dosing period corresponding to a predetermined inspection item, the comparison feature amount can be made to indicate the expected inspection data value at the determination time so as to reflect the trend of inspection data of the period of the state, in which the symptoms of the chronic disease of the subject patient are relatively calm, on the comparison feature amount. Accordingly, based on the first determination feature amount and the comparison feature amount, it is possible to appropriately determine a change at the determination time with respect to the first stable dosing period for the symptoms of the chronic disease.

In Fig. 7, an approximate straight line is calculated by applying the least square method to the specific inspection data included in the stable dosing period Tc, and the value of the approximate straight line at the determination time Tt is used as the determination time comparison feature amount Vct that is a comparison feature amount.

The determination time comparison feature amount Vct indicates an estimated value of inspection data at the determination time Tt estimated according to the trend of the comparison stable dosing period Tc. Therefore, by comparing the determination time comparison feature amount Vct with the first determination time feature amount Vt, it is possible to appropriately determine whether or not the first determination time feature amount Vt follows the trend of the comparison stable dosing period Tc. In the example shown in Fig. 7, the specific inspection data gradually decreases to show an improvement trend, but the inspection data at the determination time Tt shows a large value contrary to the trend of the specific inspection data. In a case where the estimated value of inspection data at the determination time Tt estimated according to the trend of the comparison stable dosing period Tc is set to the determination time comparison feature amount Vct, a difference between the first determination time feature amount Vt and the comparison feature amount can be clearly distinguished as in the case shown in Fig. 7. In the example shown in Fig. 7, since the determination time comparison feature amount Vct is 1220 and the first determination time feature amount Vt is 1840, Vt/Vct = 1.5 in a case where the ratio (Vt/Vct) of the first determination feature amount to the determination time comparison feature amount Vct is set as a determination parameter. Therefore, the difference between the first determination time feature amount Vt and the comparison feature amount is displayed more noticeably than Vt/Vct = 1.23 in a case where the average value Vmean 1490 of the specific inspection data is set as a comparison feature amount.

When calculating such an approximate straight line, it is preferable to calculate an approximate straight line by giving a larger weighting to specific inspection data of a period closer to the determination time. In this case, it is possible to appropriately estimate an estimated value of the inspection data at the determination time by further reflecting the trend of the inspection data of the period closer to the determination time and to set the estimated value as a comparison feature amount. Therefore, by appropriately detecting a case where the first determination feature amount is contrary to the trend of the inspection data close to the determination time by comparing the determination time comparison feature amount with the first determination feature amount, it is possible to accurately determine the abnormality of the subject patient.

In this specification, in the threshold value conditions defined in each of the first determination conditions, a comparison feature amount for statistics that is a feature amount corresponding to the comparison feature amount and a first determination feature amount for statistics corresponding to the first determination feature amount may be calculated using a plurality of pieces of inspection data of a plurality of comparison subjects in the past, a plurality of determination parameters for statistics corresponding to a determination parameter for which threshold value determination in the first determination conditions is performed may be calculated using the calculated comparison feature amount for statistics and first determination feature amount for statistics, and a value away from the average value of the plurality of calculated determination parameters for statistics by a value equal to or greater than the variation range of the determination parameters for statistics may be set as a threshold value.

In the above case, even in a case where the degree of variation is different for each piece of inspection data, it is possible to appropriately determine the abnormality of the subject patient based on the first determination conditions by suppressing a reduction in the determination accuracy of the first determination conditions due to the influence of the variation. The "comparison feature amount for statistics corresponding to the comparison feature amount" is a feature amount that is specified by the same method as the comparison feature amount using the inspection data of the same inspection item as for the comparison feature amount among a plurality of pieces of inspection data of a plurality of comparison subjects in the past. The "first determination feature amount for statistics corresponding to the first determination feature amount" is a feature amount that is specified by the same method as for the first determination feature amount using the inspection data of the same inspection item as for the first determination feature amount among a plurality of pieces of inspection data of a plurality of comparison subjects in the past.

Preferably, the comparison feature amount for statistics and the first determination feature amount for statistics are feature amounts calculated from a plurality of pieces of inspection data included in a period excluding the determination time at which there may be a change in the symptoms of the subject patient since treatment to maintain a stable state for a chronic disease for the treatment of the same disease as the subject patient is performed for a comparison subject. In this case, it is possible to calculate and use the comparison feature amount for statistics and the first determination feature amount for statistics in a period for which there is a high probability that the comparison subject is in a stable condition for the target disease. Therefore, a possibility is high that a threshold value for determining exacerbation and non-exacerbation can be appropriately set in the first determination conditions.

As an example, an example of the method of determining the threshold value of the first determination conditions will be described. In this case, for "n" patients M1 to Mn for comparison in the past for whom treatment for the same disease as the subject patient is performed, a plurality of determination parameters for statistics corresponding to the determination parameter, for which threshold value determination in the first determination conditions is performed is calculated and acquired.

As an example, a case where the determination parameter of the threshold value conditions is a ratio of inspection data at the determination time to the comparison feature amount, which is an average value of the specific inspection data of a predetermined inspection item, will be described as an example. First, a plurality of pieces of medical information are acquired for the "n" patients M1 to Mn for comparison in the past for whom treatment for the same disease as the subject patient is performed. Then, for a patient Mp (1 ≤ p ≤ n) for comparison in the past, the dosage details common period of the patient Mp for comparison is determined based on the disease associated drug corresponding to the disease of the subject patient. Then, a stable dosing period for variation calculation of the patient Mp for comparison (period corresponding to the stable dosing period of the subject patient) is determined so as to exclude a predetermined initial period and a predetermined final period, which are the same periods as those used at the time of determination of the stable dosing period of the subject patient, from the dosage details common period of the patient Mp for comparison. Then, in the stable dosing period for variation calculation of the patient Mp for comparison, "m" inspection times L1 to Lm are acquired for a predetermined inspection item. Then, for an inspection time Lq (1 ≤ q ≤ m) based on the disease associated drug of the target disease, at the time of an inspection about a comparison stable dosing period (comparison stable dosing period corresponding to inspection time Lq) that starts after the first reference time, which is before a predetermined period from the inspection time, and ends at the second reference time before the inspection time Lq after the first reference time is determined from the inspection time Lq. Then, for the predetermined inspection item, the average value of specific inspection data for statistics included in the comparison stable dosing period corresponding to the inspection time Lq is acquired as a comparison feature amount for statistics, and the inspection data of the inspection time Lq is acquired as a first determination feature amount for statistics. Then, the ratio of the first determination feature amount for statistics to the comparison feature amount for statistics is acquired as a determination parameter for statistics Uq corresponding to the determination parameter. Similarly, for the inspection times L1 to Lm, determination parameters for statistics U1 to Um corresponding to the determination parameter for the patient Mp are acquired, respectively. In the same manner, for the patients M1 to Mn, corresponding determination parameter for statistics U1' to Um' are similarly acquired, respectively.

Then, the distribution of the determination parameter values for statistics at the time of non-abnormalities is checked by calculating an average and a variance σ² from a plurality of acquired determination parameters for statistics U, and a threshold value is determined from the distribution. For example, by setting threshold value conditions based on the average value and the standard deviation σ of the distribution of the determination parameter values such that a value away from the average value by +2σ or more is determined to be abnormal, it is possible to correctly determine 97.7% of non-abnormal inspection data to be non-abnormality. Instead of the method of using the standard deviation, the number of samples of the parameters U from the smallest value of the parameters may be integrated using a histogram in which a plurality of parameters U corresponding to the determination parameters are arranged in descending order of values, and the maximum integrated value of the samples when the integrated number of samples is 97.7% of the total number of parameters U may be set as a threshold value.

As described above, the distribution of the determination parameter values for statistics corresponding to the determination parameters can be calculated using only the inspection data included in the stable dosing period for the calculation of the variation of the comparison subject. In the case of determining a threshold value based on the distribution of the calculated determination parameter values for statistics, even in a case where it is not possible to acquire the information of a period of exacerbation regarding the medical information of the comparison subject, it is possible to calculate a feature amount for statistics using the values of inspection data included in a period for which there is a high possibility that the target disease of the comparison subject has not been exacerbated. Therefore, in the first determination conditions, a possibility that a threshold value for determining abnormalities and non-abnormalities can be appropriately set is high.

In addition, in a case where the value of specific inspection data in the first stable dosing period is in a predetermined normal range, the feature amount analysis unit 13 may calculate the comparison feature amount by replacing the value of the specific inspection data with the upper limit or the lower limit of the normal range. In the case of an inspection item for which a range where the value of inspection data is determined to be normal is wide, if the first determination feature amount belongs to the normal range, there is no abnormality even if there is a difference between the comparison feature amount and the first determination feature amount. Accordingly, it is not appropriate to make a determination as abnormality. In a case where the feature amount analysis unit 13 has replaced the value of inspection data belonging to the normal range with the upper limit or the lower limit of the reference range, the comparison feature amount can be set to a value close to the upper limit or the lower limit of the reference range. Therefore, when determining an abnormality by comparing the comparison feature amount with the first determination feature amount, even in a case where the value of inspection data is widely distributed in the normal range, the abnormality of the subject patient can be accurately and easily determined by suppressing the influence of variations in the values of the specific inspection data in the normal range by determining the difference from the first determination feature amount with a value close to the upper limit or the lower limit of the reference range as the comparison feature amount. Also in a case where the value of specific inspection data in a second stable dosing period to be described later is in a predetermined normal range, it is effective that the feature amount analysis unit 13 calculates a second determination feature amount to be described later by replacing the value of specific inspection data to the upper limit or the lower limit of the normal range in order to suppress the influence of variations in the values of the specific inspection data in the normal range.

As described above, in a case where the determination section 14 includes the first determination section 14A that acquires the comparison feature amount determined from the specific inspection data included in the first stable dosing period, which is a stable dosing period before the determination time of the subject patient, and the first determination feature amount determined from the inspection data at the determination time of the subject patient and determines the presence or absence of an abnormality of the subject patient based on the acquired comparison feature amount and the acquired first determination feature amount, the first stable dosing period is a period excluding the determination time at which there may be a change in the symptoms of the subject patient since treatment to maintain a stable state for a chronic disease is performed. Accordingly, since the feature amount acquired from the inspection data included in a period of the state in which the symptoms of the chronic disease of the subject patient are relatively calm can be set as the comparison feature amount, it is possible to appropriately determine a change at the determination time with respect to the first stable dosing period for the symptoms of the chronic disease by comparing the first determination feature amount indicating the symptoms at the determination time with the comparison feature amount.

In a case where the first determination conditions are conditions in which it is determined that there is an abnormality in the first determination feature amount in a case where the ratio of the first determination feature amount to the comparison feature amount or the difference between the comparison feature amount and the first determination feature amount does not satisfy predetermined threshold value conditions, the first determination section 14A can determine the presence or absence of a change in the symptoms based on the first determination feature amount easily and appropriately on the basis of the first determination conditions.

On the other hand, in a case where the stable dosing period determined by the stable dosing period determination unit 12 is the second stable dosing period including the determination reference time at the final stage, the feature amount analysis unit 13 acquires a second determination target feature amount from inspection data corresponding to a predetermined inspection item included in the second stable dosing period Tc.

Hereinafter, an example of acquiring the second determination feature amount will be described. Fig. 8 is a diagram illustrating a method of acquiring the second determination feature amount from the second stable dosing period Tc. Here, as shown in Fig. 8, the second determination feature amount can be a coefficient of the approximate curve calculated from a plurality of pieces of specific inspection data in the second stable dosing period corresponding to a predetermined inspection item. In the example shown in Fig. 8, an approximate straight line Qc is calculated based on the specific inspection data, and the inclination of the approximate straight line (the rate of change of inspection data) is acquired as the second determination feature amount Vt2.

In addition, the second determination feature amount may be calculated based on an arbitrary inspection item as long as it is possible to determine an abnormality at the time of determination. As a method of calculating the second determination feature amount, it is possible to adopt various feature amount calculation methods according to the inspection item as long as it is possible to show the feature of specific inspection data of a desired inspection item.

In a case where the determination conditions acquired based on the composite table are second determination conditions for determining the presence or absence of an abnormality at the determination time Tt based on the second determination feature amount, when the second determination feature amount is acquired, the second determination section 14B determines whether or not the second determination conditions are satisfied based on the second determination feature amount.

For example, the second determination section 14B acquires conditions that the second determination feature amount Vt2, which is the rate of change of the specific inspection data of the inspection item KL-6, is equal to or less than the threshold value 1.1, as the second determination conditions corresponding to interstitial pneumonia and chronic exacerbation, based on the composite table. Then, the second determination section 14B acquires the second determination feature amount Vt2, which is the inclination of the approximate straight line of the specific inspection data for KL-6, and determines whether or not the conditions of Vt2 ≤ 1.1 are satisfied. Similarly, as shown in Table 4, also for the inspection items SP-D and SP-A associated with interstitial pneumonia and chronic exacerbation, the first determination section 14A determines whether or not the determination conditions defined in Table 4 are satisfied.

As described above, in a case where the determination section 14 includes the second determination section 14B that determines the presence or absence of an abnormality of the subject patient based on the second determination feature amount determined from a plurality of pieces of inspection data of the subject patient included in the second stable dosing period, which is a stable dosing period including the determination time of the subject patient at the final stage, the second stable dosing period is a period including the determination time at which there may be a change in the symptoms of the subject patient since treatment to maintain a stable state for a chronic disease is performed. Accordingly, since the second determination feature amount indicating a change in the symptoms of the chronic disease of the subject patient at the determination time and its surrounding period can be acquired from the specific inspection data included in the second stable dosing period, it is possible to appropriately determine the change in the symptoms of the chronic disease of the subject patient at the determination time and its surrounding period using the second determination feature amount.

In a case where the second determination feature amount is a coefficient of the approximate curve calculated from a plurality of pieces of specific inspection data in the second stable dosing period corresponding to a predetermined inspection item, the second determination feature amount can be made to show a change in the symptoms of the chronic disease of the subject patient at the determination time and its surrounding period. Therefore, using the second determination feature amount, it is possible to appropriately determine the change in the symptoms of the chronic disease of the subject patient at the determination time and its surrounding period.

In addition, in a case where the second determination feature amount is a coefficient of the approximate curve calculated from a plurality of specific inspection data in the second stable dosing period corresponding to a predetermined inspection item, the absolute value of the coefficient of the approximate curve may be used as a determination parameter. Alternatively, the second determination feature amount may be normalized by a predetermined reference feature amount, and predetermined threshold value conditions may be determined using the normalized second determination feature amount as a determination parameter. In this case, an appropriate value as a reference corresponding to the second determination feature amount, such as a maximum value, a minimum value, or an average value of specific inspection data, may be set as the predetermined reference feature amount. In a case where the second determination conditions are set as conditions for determining the threshold value conditions using the normalized determination parameter, it is possible to appropriately determine the presence or absence of a change in the symptoms based on the second determination feature amount by reducing the influence of a variation in the inspection data or the like.

In a case where the second determination conditions are conditions in which it is determined that there is an abnormality in the second determination feature amount in a case where the second determination feature amount does not satisfy predetermined threshold value conditions, the second determination section 14B can determine the presence or absence of a change in the symptoms based on the second determination feature amount easily and appropriately on the basis of the second determination conditions.

As described above, in a case where the feature amount analysis unit 13 acquires the determination information (composite table), in which a disease, a determination purpose, a predetermined inspection item corresponding to the disease and the determination purpose, and feature amount specifying information that is information specifying the feature amount from inspection data corresponding to the predetermined inspection item are associated with each other, and acquires a feature amount according to the feature amount specifying information corresponding to the disease of the subject patient and the desired determination purpose based on the determination information and the determination unit (the first determination section 14A or the second determination section 14B) determines the presence or absence of an abnormality at the determination time according to the determination conditions corresponding to the disease of the subject patient and the desired determination purpose based on the determination information, it is possible to determine the presence or absence of an abnormality according to the appropriate determination conditions corresponding to the disease and the determination purpose.

In addition, in a case where the determination information is information in which the period specifying information, the feature amount specifying information, and the determination conditions are associated with each other according to the combination of the disease and the determination purpose, it is possible to acquire the feature amount based on the specific inspection data of the stable dosing period that is set appropriately according to the disease. Therefore, it is possible to further improve the accuracy of abnormality determination.

In addition, the association with the determination purpose may be omitted from the composite table described above, so that the inspection item, and the feature amount specifying information, and the determination conditions are associated with each other based on the disease. In this case, the feature amount analysis unit 13 may acquire the inspection item, feature amount specifying information, and the determination information associated with the target disease based on the determination conditions. Thus, in a case where the determination information is information in which the disease, the stable dosing period, the feature amount specifying information, and the determination conditions are associated with each other, it is possible to acquire the appropriate feature amount based on the specific inspection data of the stable dosing period set appropriately according to the disease and accurately determine the presence or absence of an abnormality according to the appropriate determination conditions corresponding to the disease.

The display control unit 15 displays information obtained from each unit on the display screen of the display unit 2A when necessary. The display control unit 15 includes a first display control section 15A that displays at least a piece of information included in determination information, which will be described later, corresponding to determination on the display screen in a case where it is determined that there is an abnormality in the subject patient and a second display control section 15B that displays a stable dosing period on the display screen so as to be able to accept a change in the stable dosing period.

A medical information display screen that is display-controlled by the display control unit 15 will be described. Fig. 3 shows an example of the display screen that is controlled by the medical information processing device of the present embodiment and is displayed on the medical department terminal 2. Each field of the medical information display screen will be described with reference to Fig. 3.

As shown in Fig. 3, the medical information display screen includes a basic information display field R1 in which basic information, such as identification information (patient ID), name, age, sex, and disease of a patient, is displayed, a disease list display field R2 to display a disease from which the subject patient suffers, an inspection period display field R3 to display the presence or absence of inspection information of the subject patient along the time axis, a medical information display field R4, an inspection item display field R5, and a reference information display field R6 to be described later to displays various kinds of inspection information of the subject patient. The medical information display field R4 includes a graph display field R41 to display the inspection data of each inspection that can be graphically shown, such as a biopsy and subject inspection, in a time-series graph format at a position corresponding to the inspection time, a dosage details display field R42 showing the type of drug administered to the subject patient and a dosing period in a bar chart, and an image data display field R43 in which a thumbnail image of an image obtained by imaging the patient with an imaging apparatus, such as a CR apparatus or an MRI apparatus, is displayed at a position corresponding to the imaging date of the image.

Here, as shown in Fig. 3, the second display control section 15B displays rod-like indices Ks and Ke showing the start and end of the comparison stable dosing period TC on the medical information display screen. By receiving a user's operation of moving the indices Ks and Ke by operating an arrow indicator shown by Kc with a mouse or the like on the medical information display screen, the stable dosing period determination unit 12 of the medical information processing device 1 acquires times corresponding to the positions of Ks and Ke after the movement. Then, the stable dosing period determination unit 12 changes the time corresponding to the position of acquired Ks (or Ke) to the start (or end) of the stable dosing period Tc.

As in the example described above, in a case where the second display control section 15B displays a stable dosing period on the display screen, doctors can easily distinguish the medical information of the stable dosing period from the medical information of other periods. Accordingly, it is possible to easily understand the inspection data of the stable dosing period and the inspection data of the determination time Tt. Therefore, it is possible to help improve the efficiency of diagnosis of doctors and diagnostic accuracy.

In a case where a stable dosing period can be changed by receiving the input of the user, it is possible to determine the stable dosing period more appropriately by reflecting the determination of doctors. Therefore, it is possible to determine inspection data more accurately by determining the presence or absence of an abnormality of the determination time Tt based on the inspection data of the determined stable dosing period.

In addition, any method can be adopted as a display method of a stable dosing period, and any method can be adopted as a method of changing the stable dosing period. As a method of changing the stable dosing period, in the case of receiving the input of a period by manual operation and changing the stable dosing period based on the received period, a period that is input by receiving the input of a period from an input unit, such as a mouse or a keyboard, by adopting any graphical user interfaces (GUI) can be set as the stable dosing period. For example, the value of the stable dosing period may be directly input to a dialog from the keyboard, or the stable dosing period may be selectively input using a drop-down list or the like. In addition, any stable dosing period that can be changed by receiving the input of the user by the second display control section 2 may be used. For example, the first stable dosing period may be used, or the first stable dosing period immediately before the determination time may be used, or a comparison stable dosing period may be used, or the second stable dosing period may be used, or any combination thereof may be used.

When a determination result of the first determination section 14A is acquired, the display control unit 15 transmits the determination result to the medical department terminal 2 to display the determination result on the medical information display screen. Then, the determination result is displayed in the reference information display field R6 of Fig. 3 on the display screen of the medical department terminal 2.

In a case where one or more determination results indicate that there is an abnormality at the time of determination, the first display control section 15A displays a disease or a determination purpose associated with determination conditions corresponding to the determination that there is an abnormality (and/or determination that there is no abnormality) or at least one or more pieces of information corresponding to the determination conditions, as reference information, on the display screen based on the determination conditions shown in Table 4 in which a disease, a determination purpose, and determination conditions are associated with each other.

For example, in the reference information display field R6 of Fig. 3, a disease (interstitial pneumonia) and an inspection item (KL-6) that are associated with the determination conditions of KL-6 and the first determination feature amount (value of inspection data of KL-6: 1930) are displayed since the inspection item KL-6 does not satisfy the determination conditions as described above. In addition to this, in a case where determination corresponding to a disease and the determination purpose is performed and it is determined that there is an abnormality (in Fig. 3, there is an abnormality in alanine aminotransferase (ALT)), additional reference information is displayed in the reference information display field R6 of Fig. 3.

As described above, in a case where the first display control section 15A displays a disease or a determination purpose associated with determination conditions corresponding to the determination that there is an abnormality (and/or determination that there is no abnormality) or at least one or more pieces of information corresponding to the determination conditions, as reference information, based on the determination conditions in which a disease, a determination purpose, and determination conditions are associated with each other, it is possible to reduce the oversight of abnormalities of inspection data by presenting information, which is a clue to an abnormality or symptoms to be noted, to doctors. Therefore, it is possible to help improve the efficiency of diagnosis or diagnostic accuracy.

In a case where the first display control section 15A displays a target disease for which an abnormality has been detected, doctors can easily and quickly understand in which disease there is an abnormality in a case where the subject patient suffers from a plurality of diseases. In addition, it is preferable to perform detailed display of medical information relevant to a target disease for which there is an abnormality while displaying the target disease for which there is an abnormality, or it is preferable to display a link, a button, or the like, which is for performing detailed display of medical information relevant to a target disease for which there is an abnormality, on the display screen. In this case, the doctors can reduce the work to diagnose the symptoms with reference to various kinds of diagnostic information of the subject patient by paying attention to the target disease for which there is an abnormality. Accordingly, it is possible to perform the work efficiently.

In a case where the first display control section 15A displays an inspection item for which an abnormality has been detected, it is possible to reduce the work burden when the doctors specify and observe medical information corresponding to the inspection item. In addition, it is preferable to perform identification display of inspection data relevant to an inspection item for which there is an abnormality while displaying the inspection item for which there is an abnormality, or it is preferable to display a link, a button, or the like, which is for performing identification display of inspection data relevant to an inspection item for which there is an abnormality, on the display screen. In this case, the doctors can reduce the work to diagnose the symptoms with reference to the information of inspection data corresponding to the inspection item for which there is an abnormality. Accordingly, it is possible to perform the work efficiently.

In addition, as a display method of the determination result, it is possible to adopt any display format and any display method.

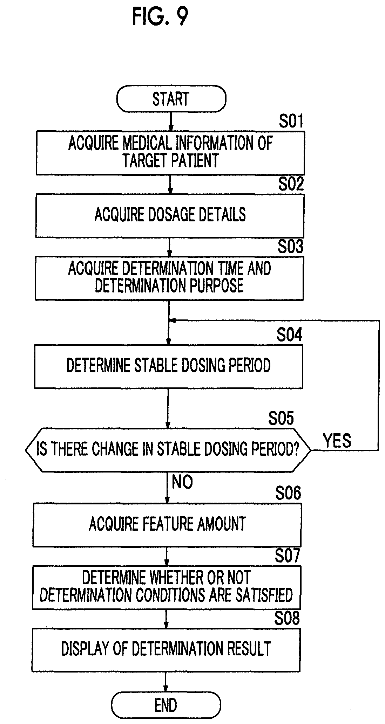

Fig. 9 is a flowchart showing the flow of the process of the medical information processing device 1 according to the first embodiment. According to Fig. 9, the flow of the process of the medical information processing device 1 according to the first embodiment will be described. Here, an example will be described in which the subject patient suffers from interstitial pneumonia that is a chronic disease and long-term therapy for the treatment of interstitial pneumonia has been performed. The interstitial pneumonia that is a chronic disease is likely to lead to death in the case of acute exacerbation. Accordingly, it is important to observe the progress by periodically performing an inspection for checking the symptoms. As inspection items of an inspection for checking the symptoms periodically, for example, KL-6, SP-A, and SP-D can be mentioned. However, inspection data of the three inspection items described above that are indices of the symptoms of interstitial pneumonia has a large variation for each patient. Accordingly, it is difficult to determine the exacerbation with a threshold value or the like determined for all patients. In addition, in the inspection data of the three inspection items described above, the value of inspection data is changed even for the same patient. Therefore, it is not appropriate to determine that the inspection data at the determination time has become worse based on the value of inspection data of only the last one time, for example. Therefore, for the three inspection items described above, even for the same patient, it is required to determine the abnormality of inspection data of the three inspection items at the determination time using the inspection data of the three inspection items at the determination time and a plurality of pieces of inspection data of inspection items corresponding to the same patient before the determination time.

First, in the medical department terminal 2, when a designation of medical information processing according to the present embodiment and the patient ID of the subject patient are input through the input unit 2B based on the user operation of doctors, the medical department terminal 2 transmits the input patient ID and the start instruction of the medical information processing to the medical information processing device 1. Then, the medical information processing device 1 receives the patient ID and the start instruction of the medical information processing and performs the medical information processing according to the present embodiment.

First, the medical information acquisition unit 16 acquires the patient ID transmitted from the medical department terminal 2, and acquires a plurality of pieces of medical information corresponding to the patient ID from the medical information management database 1A (S01).

Then, the dosage details acquisition unit 11 extracts and acquires a plurality of dosage details of the subject patient from the plurality of pieces of acquired medical information (S02). In addition, the dosage details acquisition unit 11 specifies a disease associated drug corresponding to the target disease of the subject patient based on the drug specifying information E, and acquires the dosage details corresponding to the disease associated drug selectively.

Then, the stable dosing period determination unit 12 acquires the determination time Tt and the determination purpose (S03). Then, based on the composite table, period specifying information corresponding to the disease and the determination purpose is acquired, and a stable dosing period is determined according to the acquired period specifying information and the determination time Tt (S04).

Here, based on the composite table, the first determination section 14A acquires the first reference time and the second reference time defined in Table 4 for each of the inspection items KL-6, SP-D, and SP-A based on the period specifying information corresponding to interstitial pneumonia and acute exacerbation described above, and determines a stable dosing period (the comparison stable dosing period or the second stable dosing period).

Then, the second display control section 15B displays the determined stable dosing period on the display screen. Then, the stable dosing period determination unit 12 receives and acquires an input for changing the displayed stable dosing period. In a case where there is a change in the stable dosing period (S05, Yes), the stable dosing period determination unit 12 updates the stable dosing period to the changed period and determines the changed period as a new stable dosing period (S04).

Then, in a case where there is no change in the stable dosing period (S05, No), the feature amount analysis unit 13 acquires feature amount specifying information corresponding to the disease of the subject patient, the determination purpose, and the determination time Tt based on the composite table shown in Table 4. Then, the feature amount analysis unit 13 acquires the inspection data of a predetermined inspection item included in the stable dosing period, as specific inspection data, based on the feature amount specifying information, and acquires the feature amount from the specific inspection data (S06). In a case where the feature amount specifying information acquired based on the composite table defines the first determination feature amount and the comparison feature amount, the feature amount analysis unit 13 acquires the first determination feature amount and the comparison feature amount according to the feature amount specifying information. In addition, in a case where the feature amount specifying information acquired based on the composite table defines the second determination feature amount, the feature amount analysis unit 13 acquires the second determination feature amount according to the feature amount specifying information.

Here, based on the composite table shown in Table 4, the first determination section 14A acquires the comparison feature amount and the first determination feature amount for each of the inspection items KL-6, SP-D, and SP-A based on the feature amount specifying information corresponding to interstitial pneumonia and acute exacerbation described above.

Then, the determination section 14 determines whether or not the determination conditions are satisfied based on the determination conditions acquired based on the composite table (S07). In a case where the determination conditions acquired based on the composite table are the first determination conditions, the first determination section 14A determines whether or not the first determination conditions are satisfied. In addition, in a case where the determination conditions acquired based on the composite table is the second determination conditions, the second determination section 14B determines whether or not the second determination conditions are satisfied (S07).

Here, based on the composite table, as the determination conditions corresponding to interstitial pneumonia and acute exacerbation described above, the first determination section 14A determines whether or not the determination conditions defined in Table 4 are satisfied for each of the inspection items KL-6, SP-D, and SP-A.

Then, the display control unit 15 acquires a determination result, and transmits the determination result to the medical department terminal 2 to display the determination result on the medical information display screen (S08). Then, the determination result is displayed in the reference information display field R6 of Fig. 3 on the display screen of the medical department terminal 2. In a case where a plurality of determination conditions are associated with one target disease in the determination information, determination results for the plurality of determination conditions are displayed. Of the determination results of the plurality of determination conditions, for a determination result determined to be abnormal, one or more pieces of information among the target disease name, inspection items, inspection data determined to be abnormal, and determination conditions determined to be abnormal are displayed on the display screen based on the determination information.

Here, the inspection item KL-6 for which an abnormality has been detected, the value of inspection data for which an abnormality has been detected, and the determination purpose are displayed on the reference information display field R6 of Fig. 3.

According to the first embodiment, a plurality of dosage details are acquired in which each drug administered to the subject patient and the dosing time corresponding to each drug are associated with each other, a dosage details common period equal to or longer than a predetermined period for which it can be regarded that the dosage details of the drug is common is specified based on the plurality of acquired dosage details, and a period excluding the initial stage or the final stage of the specified dosage details common period is determined as a stable dosing period. Therefore, since it is possible to determine a period for which it can be estimated that treatment to maintain a stable state for a chronic disease is performed, it is possible to provide information useful for the treatment of the chronic disease by doctors by using the medical data of the subject patient in the stable dosing period for diagnosis. As a result, it is possible to improve the diagnostic accuracy and the diagnostic efficiency.

In addition, as shown in the embodiment described above, in a case where there is provided the feature amount analysis unit 13 that extracts medical information corresponding to the inspection time included in the stable dosing period, among pieces of medical information, as specific medical information and acquires the feature amount from the specific inspection data that is inspection data corresponding to the extracted specific medical information, the feature amount indicating information regarding the symptoms of the subject patient in a period, for which it is estimated that treatment to maintain a stable state for a chronic disease is performed, is obtained. Therefore, the feature amount can be useful as reference information for the diagnosis of the subject patient.

In a case where the feature amount analysis unit 13 includes the determination section 14 that determines the presence or absence of an abnormality of the subject patient based on the feature amount as described above, it is possible to help doctors understand the subject patient easily and improve the diagnostic accuracy based on the feature amount.

The feature amount analysis unit 13 may acquire any of the comparison feature amount and the first determination feature amount first. In a case where a determination purpose, such as determination information, is not used for the processing required for each embodiment, the acquisition of the determination purpose may be omitted. In addition, the determination time acquisition processing (S03) may be performed at any timing as long as the determination time acquisition processing is performed in advance of processing that uses the determination time, such as comparison stable dosing period determination processing and first determination feature amount acquisition processing.

In the first embodiment, a configuration may be adopted in which any one of the medical information acquisition unit, the feature amount analysis unit, the determination unit, and the display control unit or any combination thereof is omitted. The determination section 14 may also be configured to include only one of the first determination section 14A and the second determination section 14B. The display control unit 15 may be configured to include only one of the first display control section 15A and the second display control section 15B, or may be configured to include neither the first display control section 15A nor the second display control section 15B.

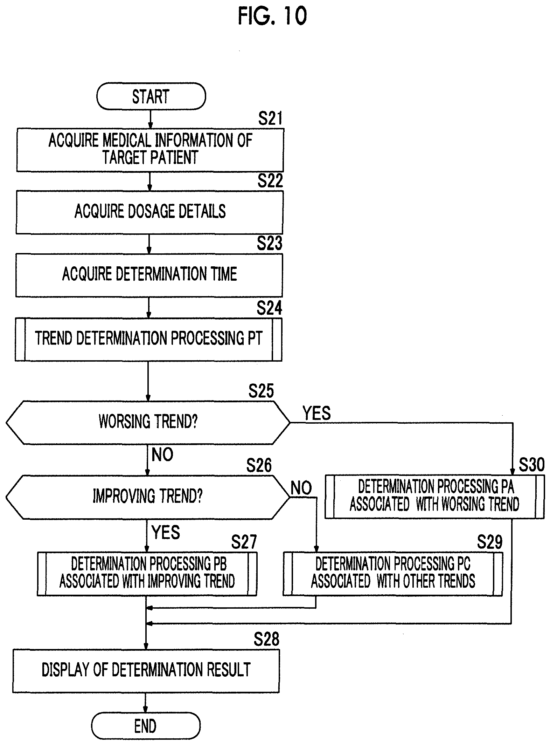

In addition, the medical information processing device 1 may appropriately adopt and apply an combination of the first determination conditions and the second determination conditions as a second embodiment. Fig. 10 is a flowchart showing the flow of the process of a medical information system in the second embodiment. Each component and the function of each component in the second embodiment are the same as those in the first embodiment except that determination processing according to the first determination conditions and determination processing according to the second determination conditions are performed in appropriate combination. Accordingly, detailed explanation thereof will be described.

According to Fig. 10, the flow of the process of the second embodiment will be described. First, the medical information acquisition unit 16 acquires the medical information of a subject patient in the same manner as in the first embodiment (S21). Then, the dosage details acquisition unit 11 extracts and acquires a plurality of dosage details of the subject patient from a plurality of pieces of medical information acquired in the same manner as in the first embodiment (S22). The stable dosing period determination unit 12 acquires the determination time Tt of the subject patient in the same manner as in the first embodiment (S23).

In the second embodiment, four determination processes (trend determination processing PT, determination processing PA, determination processing PB, determination processing PC) are set for each disease, and determination information F, which is a correspondence table in which period specifying information, feature amount specifying information, and determination conditions are associated with each other is stored in the medical information management database 1A for each determination process.

Then, the medical information processing device 1 in the second embodiment performs the trend determination processing PT (S24).

In the trend determination processing PT, the stable dosing period determination unit 12 acquires a correspondence table, and acquires period specifying information associated with the trend determination processing PT of the target disease based on the correspondence table. Here, it is assumed that the period specifying information is configured to include the first reference time and the second reference time as in the first embodiment. Then, based on the period specifying information, the stable dosing period determination unit 12 determines the second stable dosing period Tc that is a period excluding one month, which is a predetermined initial period, from the dosage details common period including the determination time and that starts after the first reference time and ends at the determination time Tt. Then, the feature amount analysis unit 13 acquires the feature amount specifying information associated with the trend determination processing PT of the target disease based on the correspondence table. Here, the feature amount specifying information is assumed to be information specifying a predetermined inspection item and a method of calculating the second determination feature amount Vt2 showing the trend of inspection data of a specific inspection item of the second stable dosing period. Then, the feature amount analysis unit 13 acquires the inclination of the approximate curve of the specific inspection data, which is the second determination feature amount Vt2, according to the feature amount specifying information associated with the trend determination processing PT. Then, the feature amount analysis unit 13 acquires the determination conditions associated with the trend determination processing PT of the target disease based on the correspondence table. Then, based on the threshold value conditions defined in the determination conditions, it is determined whether the second determination feature amount Vt2 is a worsening trend, or a improving trend, or other trends.

In a case where the second determination feature amount Vt2 is a worsening trend (S25, Yes), the medical information processing device 1 performs the determination processing PA associated with the worsening trend (S30). In a case where the second determination feature amount Vt2 is not a worsening trend (S25, No), the feature amount analysis unit 13 determines whether or not the second determination feature amount Vt2 is a improving trend based on additional threshold value conditions included in the determination conditions associated with the trend determination processing PT of the target disease (S26). In a case where the second determination feature amount Vt2 is a improving trend (S26, Yes), the medical information processing device 1 performs the determination processing PB associated with the improving trend (S27). In a case where the second determination feature amount Vt2 is neither a improving trend nor a worsening trend (S26, No), the medical information processing device 1 performs the determination processing PC associated with other trends (S29).

The determination processing PA includes processing in which the stable dosing period determination unit 12 acquires period specifying information associated with the determination processing PA of the target disease based on the correspondence table and determines a predetermined second stable dosing period according to the acquired period specifying information (the second stable dosing period may be the same as the second stable dosing period of the trend determination processing PT (S24), or may be different from the second stable dosing period of the trend determination processing PT (S24)), processing in which the feature amount analysis unit 13 acquires feature amount specifying information associated with the determination processing PA of the target disease based on the correspondence table and determines the second determination feature amount Vt2 according to the acquired feature amount specifying information (the second determination feature amount may be the same as the second determination feature amount of the trend determination processing PT (S24), or may be different from the second determination feature amount of the trend determination processing PT (S24)), and processing in which the second determination section 14B acquires determination conditions associated with the determination processing PA based on the correspondence table and determines whether or not the second determination conditions are satisfied according to the acquired second determination conditions.

Here, as described in the example shown in Fig. 8, the second determination conditions are defined to be conditions for determining that there is an abnormality in a case where the second determination feature amount, which is the absolute value of the inclination of the approximate straight line of specific inspection data included in the second stable dosing period is larger than predetermined threshold value conditions. According to the second determination conditions, it is possible to appropriately detect that the symptoms of the chronic disease have become gradually worse. Therefore, it is possible to appropriately perform abnormality determination by performing the determination processing PA based on the second determination conditions. It is preferable to apply any determination conditions, by which it is possible to determine the occurrence of a change in the symptoms of the chronic disease caused in a case where the symptoms have become worse, to the determination processing PA. In addition, as shown in the example of Fig. 5, the method based on the conditions in which the comparison feature amount Vc is an average value of a plurality of pieces of specific inspection data in the first stable dosing period Tc corresponding to a predetermined inspection item, the first determination feature amount Vt is inspection data of determination, and it is determined that there is an abnormality in a case where the ratio Vt/Vc of the first determination feature amount to the comparison feature amount does not satisfy third threshold value conditions is somewhat unsuitable for the detection of a case where the symptoms become gradually worse in a narrow numerical range.

The determination processing PB includes processing in which the stable dosing period determination unit 12 acquires period specifying information associated with the determination processing PB of the target disease based on the correspondence table and determines a comparison stable dosing period that is a predetermined first stable dosing period according to the acquired period specifying information, processing in which the feature amount analysis unit 13 acquires feature amount specifying information associated with the determination processing PB of the target disease based on the correspondence table and determines a comparison feature amount and the first determination feature amount Vt1 according to the acquired feature amount specifying information, and processing in which the first determination section 14A acquires eleventh determination conditions associated with the determination processing PB based on the correspondence table and determines whether or not the eleventh determination conditions are satisfied according to the acquired eleventh determination conditions.

Here, as described in the example shown in Fig. 7, the eleventh determination conditions are assumed to be conditions in which it is determined, based on the approximate curve Qc calculated from a plurality of pieces of specific inspection data in the comparison stable dosing period Tc corresponding to a predetermined inspection item, that there is an abnormality in a case where the difference between the first determination feature amount Vt and the determination time comparison feature amount Vct, which is a value of the approximate curve corresponding to the determination time Tt, or the ratio between the determination time comparison feature amount Vct and the first determination feature amount Vt does not satisfy the second threshold value conditions. Thus, the method of determining an abnormality according to the difference between the determination time comparison feature amount and the first determination feature amount is suitable for determining the abnormality of specific inspection data contrary to the trend in which the symptoms of the subject patient are improving. Therefore, it is possible to perform abnormality determination more accurately by performing the determination processing PB based on the first determination conditions when the symptoms of the subject patient are improving.

The determination processing PC includes processing in which the stable dosing period determination unit 12 acquires period specifying information associated with the determination processing PC of the target disease based on the correspondence table and determines a comparison stable dosing period that is a predetermined first stable dosing period according to the acquired period specifying information, processing in which the feature amount analysis unit 13 acquires feature amount specifying information associated with the determination processing PC of the target disease based on the correspondence table and determines a comparison feature amount and the first determination feature amount Vt1 according to the acquired feature amount specifying information, and processing in which the first determination section 14A acquires twelfth determination conditions associated with the determination processing PC based on the correspondence table and determines whether or not the twelfth determination conditions are satisfied according to the acquired twelfth determination conditions.

Here, as described in the example shown in Fig. 5, the twelfth determination conditions are assumed to be conditions for determining that there is an abnormality in a case where the ratio between the comparison feature amount Vc, which represents an average value of a plurality of pieces of specific inspection data in the comparison stable dosing period Tc corresponding to a predetermined inspection item, and the first determination feature amount Vt does not satisfy predetermined threshold value conditions. Thus, in the case of using the first determination conditions for determining an abnormality according to the difference between the first determination feature amount and the comparison feature amount indicating the average value of specific inspection data, wide applications to the determination of the symptoms of the subject patient can be made even in a case where there is a temporal variation in the inspection time of specific inspection data in the stable dosing period or the number of pieces of specific inspection data is small. Therefore, in a case where the symptoms of the subject patient are neither improving nor exacerbated, it is possible to appropriately perform abnormality determination by performing the determination processing PC based on the twelfth determination conditions described above. In the twelfth determination conditions, even if the difference between the comparison feature amount Vc and the first determination feature amount Vt is used instead of the ratio of the first determination feature amount Vt to the comparison feature amount Vc, the same effect is obtained.

As in the first embodiment, the display control unit 15 transmits an instruction to display the determination result on the display screen to the medical department terminal 2 and ends the process of the second embodiment (S28).

According to the third embodiment, in the trend analysis processing, the trend of the symptoms is checked based on the desired inspection data of the subject patient, and determination processing suitable for each trend is performed. Therefore, it is possible to accurately determine the abnormality of the subject patient. As determination conditions corresponding to the improving trend, determination conditions corresponding to the worsening trend, and determination conditions corresponding to other trends, any determination conditions may be applied as long as these are suitable for each trend. The trend of the symptoms may be classified using other category classification methods, such as exacerbation and others. In this case, determination conditions suitable for the other category classification may be set so as to be associated with each other.

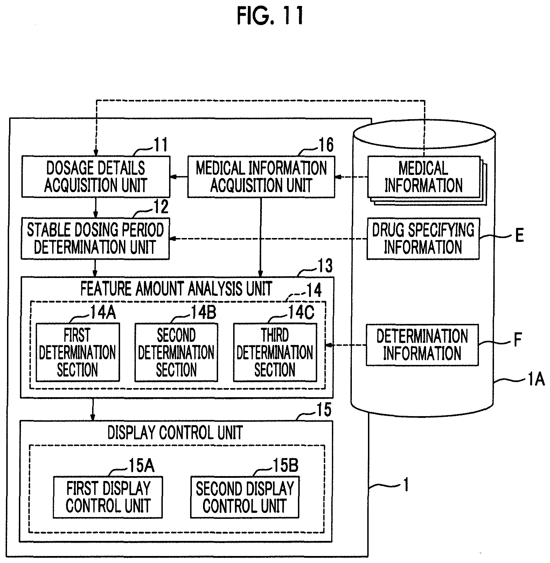

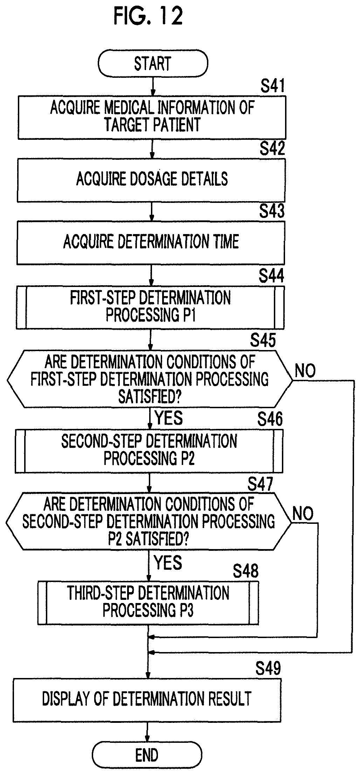

In addition, a third embodiment will be described. Fig. 11 is a block diagram showing the schematic configuration of a medical information processing device according to the third embodiment. Fig. 12 is a flowchart illustrating the flow of the process of a medical information system using the medical information processing device according to the third embodiment. The third embodiment is different from the first embodiment in that the determination section 14 includes a third determination section 14C and the third determination section 14C determines whether or not third determination conditions for determining the presence or absence of an abnormality at the determination time are satisfied. Other than these, each component and the function of each component in the third embodiment are the same as those in the first embodiment. Accordingly, explanation of common portions will be omitted.

According to Fig. 12, the flow of the process of the third embodiment will be described. First, as in the first embodiment, the medical information acquisition unit 16 acquires the medical information of the subject patient (S41). Then, the dosage details acquisition unit 11 extracts and acquires a plurality of dosage details of the subject patient from a plurality of pieces of medical information acquired in the same manner as in the first embodiment (S42). The stable dosing period determination unit 12 acquires the determination time Tt of the subject patient in the same manner as in the first embodiment (S43).

In the third embodiment, determination processes of three steps (first-step determination processing P1, second-step determination processing P2, and third-step determination processing P3) are set for each disease, and a correspondence table in which period specifying information, feature amount specifying information, and determination conditions are associated with each other is stored in the medical information management database 1A for each determination process.

Then, the medical information processing device 1 in the third embodiment performs the first-step determination processing P1 (S44).

In the first-step determination processing P1, the third determination section 14C acquires a correspondence table, and acquires inspection data immediately before the determination time and inspection data at the determination time, which correspond to a predetermined inspection item associated with the first-step determination processing P1 of the target disease, based on the correspondence table. Then, the third determination section 14C acquires the third determination conditions based on the correspondence table. The third determination conditions are conditions in which it is determined that there is no abnormality in a case where the ratio of the inspection data at the determination time to the inspection data immediately before the determination time is equal to or less than a predetermined threshold value. For example, the third determination conditions may be conditions in which it is determined that there is no abnormality in a case where the difference between the inspection data at the determination time and the inspection data immediately before the determination time is equal to or less than a predetermined threshold value. Then, the third determination section 14 determines whether or not the ratio of the inspection data at the determination time to the inspection data immediately before the determination time satisfies the defined threshold value conditions according to the third determination conditions.

In a case where it is determined that the determination conditions of the first-step determination processing P1 are satisfied, (S45, Yes), the medical information processing device 1 performs the second-step determination processing P2 (S46).

In the second-step determination processing P2, the stable dosing period determination unit 12 acquires a correspondence table, and acquires period specifying information associated with the second-step determination processing P2 of the target disease based on the correspondence table. Here, it is assumed that the period specifying information is configured to include the first reference time and the second reference time as in the first embodiment. Then, based on the period specifying information, the stable dosing period determination unit 12 determines a stable dosing period that is a period excluding one month, which is a predetermined initial period, from the dosage details common period, determines a first stable dosing period of the stable dosing period, and determines a first stable dosing period immediately before the determination time Tt of the first stable dosing period. Based on the first stable dosing period, the stable dosing period determination unit 12 determines the comparison stable dosing period Tc that starts after the first reference time and ends at the second reference time before the determination time Tt. Then, the feature amount analysis unit 13 acquires the feature amount specifying information associated with the second-step determination processing P2 of the target disease based on the correspondence table. Here, the feature amount specifying information is assumed to be information that specifies a predetermined inspection item, the comparison feature amount Vc obtained from the inspection data of a specific inspection item of the comparison stable dosing period, and a method of calculating the first determination feature amount Vt1 showing the value of inspection data of the specific inspection item at the determination time. Then, the feature amount analysis unit 13 acquires the average value of the specific inspection data, which is the first determination feature amount Vt1 and the comparison feature amount Vc, according to the feature amount specifying information associated with the second-step determination processing P2. Then, the feature amount analysis unit 13 acquires the determination conditions associated with the second-step determination processing P2 of the target disease based on the correspondence table. Then, based on the threshold value conditions defined in the determination conditions, the first determination section 14A determines whether or not first conditions that the ratio of the first determination feature amount Vt1 to the comparison feature amount Vc is equal to or less than a predetermined threshold value are satisfied.

In a case where it is determined that the determination conditions of the second-step determination processing P2 are satisfied, (S47, Yes), the medical information processing device 1 performs the third-step determination processing P3 (S48).

In the third-step determination processing P3, the stable dosing period determination unit 12 acquires a correspondence table, and acquires period specifying information associated with the third-step determination processing P3 of the target disease based on the correspondence table. Here, it is assumed that the period specifying information is configured to include the first reference time and the second reference time as in the first embodiment. Then, based on the period specifying information, the stable dosing period determination unit 12 determines a period excluding one month, which is a predetermined initial period, from the dosage details common period as a stable dosing period, and determines the second stable dosing period Tc that starts after the first reference time and ends at the determination time Tt. Then, the feature amount analysis unit 13 acquires the feature amount specifying information associated with the third-step determination processing P3 of the target disease based on the correspondence table. Here, the feature amount specifying information is assumed to be information that specifies a predetermined inspection item and a method of calculating the second determination feature amount Vt2 showing the trend of inspection data of a specific inspection item of the second stable dosing period. Then, the feature amount analysis unit 13 acquires the inclination of the approximate curve of the specific inspection data as the second determination feature amount Vt2 according to the feature amount specifying information associated with the third-step determination processing P3. Then, the feature amount analysis unit 13 acquires the determination conditions associated with the third-step determination processing P3 of the target disease based on the correspondence table. Then, based on the threshold value conditions defined in the determination conditions, the second determination section 14B determines whether or not second conditions that the second determination feature amount Vt2 is equal to or less than a predetermined threshold value are satisfied.

In a case where the third-step determination processing P3 is completed, the display control unit 15 displays a determination result (S49). Similarly, the display control unit 15 displays a determination result even in a case where it is determined that the determination conditions of the first-step determination processing P1 are not satisfied (S45, No) and a case where it is determined that the determination conditions of the second-step determination processing P2 are not satisfied (S47, No) (S49).

According to the third embodiment, in the first-step determination processing, by determining whether or not the third determination conditions are satisfied in which it is determined that there is no abnormality in a case where the ratio of the inspection data at the determination time to the inspection data immediately before the determination time (absolute value of the ratio) or the difference between the inspection data at the determination time and the inspection data immediately before the determination time (absolute value of the difference) is equal to or less than a predetermined threshold value, an abnormality is determined first using a simple method with a low detection load. Then, only in a case where an abnormality has been detected in the first-step determination processing, other determination processes from the second-step determination processing are performed. Therefore, it is possible to accurately determine the presence or absence of an abnormality while reducing the calculation load. In the second-step determination processing P2 and the third-step determination processing P3, appropriate determination conditions that are different from those in the first-step determination processing can be arbitrarily adopted. The third-step determination processing P3 may be omitted. In addition to the third-step determination processing P3, additional determination processing may be performed by the arbitrary number of times. In the determination processing of each step, any determination conditions can be adopted by arbitrarily combining appropriate determination processes that are different from those in the first-step determination processing. For example, for determination processes from the second-step determination processing, various kinds of first determination conditions and second determination processing conditions may be adopted by the arbitrary number of times in any combination in the determination processing of each step as long as different determination conditions are used.

Also in the second and third embodiments, it is preferable that the display control unit 15 includes the first display control section 15A so that reference information relevant to the determination conditions corresponding to abnormality determination is displayed on the display screen in a case where any one or more of determination results indicate that there is an abnormality at the determination time. In addition, also in the second and third embodiments, it is preferable that the display control unit 15 includes the second display control section 15B that displays a determined stable dosing period on the display screen. In this case, the stable dosing period determination unit 12 receives and acquires an input for changing a stable dosing period displayed in the same manner as in the first embodiment, and updates the stable dosing period to the changed period and determines the changed period as a new stable dosing period in a case where there is a change in the stable dosing period.

The threshold value conditions in this specification may be arbitrarily set for each inspection item based on the findings in the medical diagnosis if the value of the medical data is a level that should be noted. The threshold value conditions shown in each embodiment are assumed to determine an abnormality in the case of the threshold value or more in a case where the possibility of an abnormality increases as a value used in the determination increases and to determine an abnormality in the case of the threshold value or less in a case where the possibility of an abnormality increases as the value used in the determination decreases. In addition, for each disease, even for the same inspection item, a numerical range determined to be an abnormal value may be made different. In this case, for example, acquiring the target disease of the subject patient and changing the numerical range of the abnormal value applied to the medical data of the subject patient according to the target disease can be considered. In addition, depending on the determination purpose, even for the same inspection item, a numerical range determined to be an abnormal value may be made different.

The invention is not limited to the present embodiment, and some or all of the components of the medical information processing device may be formed by one workstation, or may be formed by one or more workstations, servers, and storage devices that are connected to each other through a network. Each device is controlled by a program for performing the medical information display of this specification, which is installed from a recording medium, such as a CD-ROM. In addition, the program may be installed after being downloaded from the storage device of a server connected through a network, such as the Internet.

In addition, each of the embodiments described above is for illustrative purposes, and any modification and application may be arbitrarily made without departing from the scope of the present invention.

## Claims

1. A medical information processing device, comprising:
a dosage details acquisition unit (11) configured to acquire a plurality of dosage details in which information specifying a drug administered to a subject patient, an amount of the drug, and a dosing date of the drug are associated with each other; and
a stable dosing period determination unit (12) configured to identify a dosing period equal to or longer than a predetermined period according to a target disease and a determination purpose, among dosing periods of the drug for which it is regarded that both the information specifying the drug and the amount of the drug are the same, based on the plurality of acquired dosage details, to specify the identified dosing period as a dosage details common period, and to determine a period excluding a predetermined initial or final period from the specified dosage details common period as a stable dosing period;
a medical information acquisition unit (16) configured to acquire medical information including an item of inspections performed on the subject patient, inspection data of the subject patient corresponding to each inspection item, and an inspection time of the inspection data; and
a feature amount analysis unit (13) configured to extract the medical information corresponding to the inspection item that is performed at the inspection time during the stable dosing period, from the acquired medical information, as specific medical information and to acquire a feature amount from specific inspection data that is the inspection data corresponding to the extracted specific medical information,
wherein the feature amount analysis unit (13) further includes a determination section configured to acquire a determination time of the subject patient, and to determine presence or absence of an abnormality with respect to the determination purpose of the target disease at the determination time according to determination conditions corresponding to the determination purpose of the target disease based on the feature amount extracted from the specific inspection data included in the stable dosing period before the determination time.

2. The medical information processing device according to claim 1,
wherein the dosage details acquisition unit (11) is adapted to acquire a disease of the subject patient as the target disease, and to select and to acquire the dosage details corresponding to a disease associated drug, which is known to be administered for the target disease, among the acquired dosage details of the subject patient.

3. The medical information processing device according to claim 1,
wherein the feature amount analysis unit (13) is adapted to acquire determination information in which a disease, a first inspection item that is a predetermined inspection item corresponding to the disease, feature amount specifying information that is information specifying the feature amount from the inspection data corresponding to the first inspection item, and the determination conditions corresponding to this feature amount specifying information are associated with each other, and to acquire the feature amount according to the feature amount specifying information corresponding to the disease of the subject patient based on the determination information, and
the determination section (14) is adapted to determine presence or absence of an abnormality at the determination time according to the determination conditions corresponding to the feature amount specifying information based on the acquired determination information.

4. The medical information processing device according to claim 3, further comprising:
a first display control unit (15A) for displaying at least a piece of information included in the determination information corresponding to the determination, on a display screen, in a case where it is determined that there is an abnormality at the determination time based on the determination information.

5. The medical information processing device according to any one of claims 1, 3 and 4,
wherein the stable dosing period determination unit (12) is adapted to determine a first stable dosing period immediately before the determination time among first stable dosing periods that are the stable dosing periods before the determination time,
the feature amount analysis unit is adapted to acquire a comparison feature amount, which is the feature amount determined from the specific inspection data of the first stable dosing period immediately before the determination time, and a first determination feature amount, which is a feature amount corresponding to the comparison feature amount determined from the inspection data of the subject patient at the determination time, and
the determination section (14) includes a first determination section (14A) for determining whether or not first determination conditions for determining presence or absence of an abnormality at the determination time based on the comparison feature amount and the first determination feature amount are satisfied.

6. The medical information processing device according to claim 5,
wherein the stable dosing period determination unit (12) is further adapted to determine a period having a start time after a first reference time that is a predetermined period before the determination time and having an end time before a second reference time that is after the start time and before an additional predetermined period from the determination time, among the first stable dosing periods immediately before the determination time, as a comparison stable dosing period, and
the feature amount analysis unit (13) is adapted to determine the comparison feature amount from the specific inspection data included in the comparison stable dosing period.

7. The medical information processing device according to claim 6,
wherein the stable dosing period determination unit (12) is adapted to acquire period specifying information in which a disease and the first reference time and the second reference time are associated with each other, to acquire the first reference time and the second reference time corresponding to a target disease, which is a disease of the subject patient, based on the period specifying information, and to determine the comparison stable dosing period based on the first reference time and the second reference time.

8. The medical information processing device according to any one of claims 5 to 7,
wherein the feature amount analysis unit (13) is adapted to acquire the comparison feature amount using a predetermined number of pieces of the specific inspection data going back into a past in time series from an end of the first stable dosing period immediately before the determination time.

9. The medical information processing device according to any one of claims 5 to 8,
wherein the comparison feature amount is an average value of a plurality of pieces of the specific inspection data in the first stable dosing period corresponding to a second inspection item that is a predetermined inspection item.

10. The medical information processing device according to any one of claims 5 to 8,
wherein the comparison feature amount is a value of an approximate curve corresponding to the determination time in the approximate curve calculated from a plurality of pieces of the specific inspection data in the first stable dosing period corresponding to a third inspection item that is a predetermined inspection item.

11. The medical information processing device according to any one of claims 5 to 9,
wherein, in the first determination conditions, it is determined that there is an abnormality at the determination time in a case where a difference between the comparison feature amount and the first determination feature amount or a ratio between the comparison feature amount and the first determination feature amount does not satisfy first threshold value conditions.

12. The medical information processing device according to claim 11,
wherein, in the first threshold value conditions, a comparison feature amount for statistics that is a feature amount corresponding to the comparison feature amount and a first determination feature amount for statistics corresponding to the first determination feature amount are calculated using a plurality of pieces of inspection data of a plurality of comparison subjects in a past, a plurality of determination parameters for statistics corresponding to a determination parameter for which threshold value determination in the first determination conditions is performed are calculated using the calculated comparison feature amount for statistics and first determination feature amount for statistics, and a value away from an average value of the plurality of calculated determination parameters for statistics by a value equal to or greater than a variation range of the determination parameters for statistics is set as a threshold value.

13. The medical information processing device according to any one of claims 5 to 12,
wherein, in a case where a value of the specific inspection data in the first stable dosing period is in a predetermined normal range, the feature amount analysis unit (13) is adapted to calculate the comparison feature amount by replacing the value of the specific inspection data with an upper limit or a lower limit of the normal range.

14. The medical information processing device according to any one of claims 1 and 3 to 13,
wherein the stable dosing period determination unit (12) is adapted to determine a second stable dosing period that is the stable dosing period including the determination time in a final stage,
the feature amount analysis unit (13) is adapted to acquire a second determination feature amount from a plurality of pieces of the inspection data of the subject patient in the second stable dosing period, and
the determination section (14) further includes a second determination section (14B) for determining whether or not second determination conditions for determining presence or absence of an abnormality at the determination time based on the second determination feature amount are satisfied.

15. The medical information processing device according to claim 14,
wherein the second determination feature amount is a coefficient of an approximate curve calculated from a plurality of pieces of the specific inspection data in the second stable dosing period corresponding to a fourth inspection item that is a predetermined inspection item.

16. The medical information processing device according to claim 15,
wherein, in the second determination conditions, it is determined that there is an abnormality at the determination time in a case where the second determination feature amount does not satisfy second threshold value conditions.

17. The medical information processing device according to any one of claims 14 to 16,
wherein the determination section (14) is adapted to classify the second determination feature amount into any one of a worsening trend, an improving trend, and other trends based on the second determination feature amount,
the second determination section (14B) is adapted to determine whether or not the second determination conditions are satisfied in a case where the second determination feature amount is a worsening trend,
the first determination section (14A) is adapted to determine whether or not the first determination conditions are satisfied in a case where the second determination feature amount is an improving trend,
the first determination section (14A) is adapted to determine whether or not the additional first determination conditions are satisfied in a case where the second determination feature amount is other trends,
the first determination conditions are conditions in which the comparison feature amount is a determination time comparison feature amount, which is a value of an approximate curve corresponding to the determination time that is obtained based on the approximate curve calculated from a plurality of pieces of the inspection data corresponding to a fifth inspection item that is a predetermined inspection item in the first stable dosing period, and in which it is determined that there is an abnormality in a case where a difference between the determination time comparison feature amount and the first determination feature amount or a ratio between the determination time comparison feature amount and the first determination feature amount does not satisfy third threshold value conditions, and
the additional first determination conditions are conditions in which the comparison feature amount is an average value of a plurality of pieces of the inspection data corresponding to a sixth inspection item that is a predetermined inspection item in the first stable dosing period and in which it is determined that there is an abnormality at the determination time in a case where a difference between the average value and the first determination feature amount or a ratio between the average value and the first determination feature amount does not satisfy fourth threshold value conditions.

18. The medical information processing device according to any one of claims 1 to 17, further comprising:
a second display control unit (15B) is adapted to display the stable dosing period on a display screen,
wherein the stable dosing period determination unit (12) is adapted to receive and to acquire an input to change the displayed stable dosing period and changes the stable dosing period.

19. The medical information processing device according to any one of claims 2 to 18,
wherein the dosage details acquisition unit (11) is adapted to acquire drug specifying information in which each disease is associated with the disease associated drug, which is known to be administered for the disease, and to select and to acquire the dosage details corresponding to the disease associated drug corresponding to the target disease based on the acquired drug specifying information.

20. The medical information processing device according to any one of claims 6 to 19,
wherein the determination section (14) further includes a third determination section for determining whether or not third determination conditions for determining presence or absence of an abnormality at the determination time based on a difference between a value of the inspection data corresponding to a seventh inspection item that is a predetermined inspection item at the determination time and a value of inspection data corresponding to the seventh inspection item immediately before the determination time or a ratio between a value of the inspection data corresponding to an eighth inspection item that is a predetermined inspection item at the determination time and a value of inspection data corresponding to the eighth inspection item immediately before the determination time are satisfied, and
in the determination section (14), the third determination section is adapted to determine whether or not the third determination conditions are satisfied, and the first determination section is adapted to determine presence or absence of an abnormality at the determination time according to the first determination conditions only in a case where it is determined that there is no abnormality based on the third determination conditions.

21. The medical information processing device according to any one of claims 14 to 19,
wherein the determination section (14) further includes a third determination section for determining whether or not third determination conditions for determining presence or absence of an abnormality at the determination time based on a difference between a value of the inspection data corresponding to a ninth inspection item that is a predetermined inspection item at the determination time and a value of inspection data corresponding to the ninth inspection item immediately before the determination time or a ratio between a value of the inspection data corresponding to a ninth inspection item that is a predetermined inspection item at the determination time and a value of inspection data corresponding to the ninth inspection item immediately before the determination time are satisfied, and
in the determination section (14), the third determination section is adapted to determine whether or not the third determination conditions are satisfied, and the second determination section is adapted to determine presence or absence of an abnormality at the determination time according to the second determination conditions only in a case where it is determined that there is no abnormality based on the third determination conditions.

22. A medical information processing method executed by a medical information processing device, comprising:
a dosage details acquisition step of acquiring a plurality of dosage details in which information specifying a drug administered to a subject patient, an amount of drug, and a dosing date of the drug are associated with each other; and
a stable dosing period determination step of identifying a dosing period equal to or longer than a predetermined period according to a target disease and a determination purpose, among dosing periods of the drug for which it is regarded that both information specifying the drug and the amount of drug are the same, based on the plurality of acquired dosage details, specifying the identified dosing period as a dosage details common period, and determining a period excluding a predetermined initial or final period from the specified dosage details common period as a stable dosing period,
acquiring medical information including an item of inspections performed on the subject patient, inspection data of the subject patient corresponding to each inspection item, and an inspection time of the inspection data; and
extracting the medical information corresponding to the inspection item that is performed at the inspection time during the stable dosing period, from the acquired medical information, as specific medical information and acquires a feature amount from specific inspection data that is the inspection data corresponding to the extracted specific medical information,
acquiring a determination time of the subject patient, and determining presence or absence of an abnormality with respect to the determination purpose of the target disease at the determination time according to determination conditions corresponding to the determination purpose of the target disease based on the feature amount extracted from the specific inspection data included in the stable dosing period before the determination time.

23. A computer-readable recording medium having stored therein a medical information processing program causing a computer to execute the steps of claim 22.

## Patentansprüche

1. Verarbeitungsvorrichtung für medizinische Information, umfassend:
eine Dosierangaben-Erfassungseinheit (11), konfiguriert zum Erfassen mehrerer Dosierangaben, in denen Information, die eine einem Patienten verabreichte Arznei spezifiziert, eine Menge der Arznei und ein Dosierdatum der Arznei miteinander in Verbindung stehen; und
eine Stabile-Dosierzeitspannen-Bestimmungseinheit (12), konfiguriert zum Identifizieren einer Dosierzeitspanne gleich oder länger als eine vorbestimmte Zeitspanne nach Maßgabe einer Zielkrankheit, und eines Bestimmungszwecks, unter Dosierzeitspannen der Arznei, für die angenommen wird, dass sowohl die die Arznei spezifizierende Information als auch die Menge der Arznei gleich sind, basierend auf den mehreren erfassten Dosierangaben, um die identifizierte Dosierzeitspanne als eine gemeinsame Dosierangabenzeitspanne zu identifizieren, und um als eine stabile Dosierzeitspanne eine Zeitspanne zu bestimmen, die eine vorbestimmte Anfangs- oder Endzeitspanne von der spezifizierten gemeinsamen Dosierangabenzeitspanne ausschließt;
eine Medizininformations-Erfassungseinheit (16), konfiguriert zum Erfassen von medizinischer Information einschließlich eines Gegenstands von an dem Patienten vorgenommenen Untersuchungen, Untersuchungsdaten des Patienten entsprechend jedem Untersuchungsgegenstands, und Untersuchungszeit der Untersuchungsdaten; und
eine Merkmalsgrößen-Analysiereinheit (13), konfiguriert zum Extrahieren der medizinischen Information entsprechend dem Gegenstand der Untersuchung, die zu der Untersuchungszeit während der stabilen Dosierzeitspanne ausgeführt wird, aus der erfassten medizinischen Information als spezifische medizinische Information, und zum Erfassen einer Merkmalsgröße aus den spezifischen Untersuchungsdaten, das sind die Untersuchungsdaten entsprechend der extrahierten spezifischen medizinischen Information,
wobei die Merkmalsgrößen-Analysiereinheit (13) weiterhin einen Bestimmungsabschnitt aufweist, konfiguriert zum Erfassen einer Bestimmungszeit des Patienten und zum Bestimmen des Vorhandenseins oder des Fehlens einer Anormalität bezüglich des Bestimmungszwecks der Zielkrankheit zu der Bestimmungszeit nach Maßgabe von Bestimmungsbedingungen entsprechend dem Bestimmungszweck der Zielkrankheit, basierend auf der aus den spezifischen Untersuchungsdaten extrahierten Merkmalsgröße innerhalb der stabilen Dosierzeitspanne vor der Bestimmungszeit.

2. Verarbeitungsvorrichtung nach Anspruch 1,
bei der die Dosierangaben-Erfassungseinheit (11) ausgebildet ist zum Erfassen einer Krankheit des Patienten als die Zielkrankheit, und zum Auswählen und zum Erfassen der Dosierangaben entsprechend einer Krankheit in Verbindung mit einer Arznei, von der bekannt ist, dass sie für die Zielkrankheit zu verabreichen ist, unter den erfassten Dosierangaben des Patienten.

3. Verarbeitungsvorrichtung nach Anspruch 1,
bei der die Merkmalsgrößen-Analysiereinheit (13) dazu ausgebildet ist, Bestimmungsinformation zu erfassen, in der eine Krankheit, ein erster Untersuchungsgegenstand, der ein vorbestimmter Untersuchungsgegenstand entsprechend der Krankheit ist, Merkmalsgrößen-Spezifizierinformation, das ist Information, die die Merkmalsgröße spezifiziert aus den Untersuchungsdaten entsprechend dem ersten Untersuchungsgegenstand, und die Bestimmungsbedingungen entsprechend dieser Merkmalsgrößen-Spezifizierinformation, miteinander in Verbindung stehen, und zum Erfassen der Merkmalsgröße gemäß der Merkmalsgrößen-Spezifizierinformation entsprechend der Krankheit des Patienten, basierend auf der Bestimmungsinformation, und
der Bestimmungsabschnitt (14) dazu ausgebildet ist, das Vorhandensein oder das Fehlen einer Anormalität zu der Bestimmungszeit gemäß den Bestimmungsbedingungen entsprechend der Merkmalsgrößen-Spezifizierinformation basierend auf der erfassten Bestimmungsinformation zu bestimmen.

4. Verarbeitungsvorrichtung nach Anspruch 3, weiterhin umfassend:
eine erste Anzeigesteuereinheit (15A) zum Anzeigen mindestens eines Informationsstücks, das in der der Bestimmung entsprechenden Bestimmungsinformation enthalten ist, auf einem Anzeigebildschirm, falls anhand der Bestimmungsinformation bestimmt wird, dass es zu der Bestimmungszeit eine Anormalität gibt.

5. Verarbeitungsvorrichtung nach einem der Ansprüche 1, 3 und 4,
bei der die Stabile-Dosierzeitspannen-Bestimmungseinheit (12) dazu ausgebildet ist, eine erste stabile Dosierzeitspanne unmittelbar vor der Bestimmungszeit unter ersten stabilen Dosierzeitspannen zu bestimmen, bei denen es sich um die stabilen Dosierzeitspannen vor der Bestimmungszeit handelt,
die Merkmalsgrößen-Analysiereinheit dazu ausgebildet ist, eine Vergleichs-Merkmalsgröße zu erfassen, bei der es sich um die Merkmalsgröße handelt, die aus den spezifischen Untersuchungsdaten der ersten stabilen Dosierzeitspanne unmittelbar vor der Bestimmungszeit handelt, und eine erste Bestimmungs-Merkmalsgröße zu erfassen, bei der es sich um eine Merkmalsgröße entsprechend der Vergleichs-Merkmalsgröße handelt, bestimmt anhand der Untersuchungsdaten des Patienten zu der Bestimmungszeit, und
der Bestimmungsabschnitt (14) einen ersten Bestimmungsabschnitt (14A) enthält, um zu bestimmen, ob erste Bestimmungsbedingungen zum Bestimmen des Vorhandenseins oder des Fehlens einer Anormalität zu der Bestimmungszeit basierend auf der Vergleichs-Merkmalsgröße und der ersten Bestimmungs-Merkmalsgröße erfüllt sind.

6. Verarbeitungsvorrichtung nach Anspruch 5,
bei der die Stabile-Dosierzeitspannen-Bestimmungseinheit (12) weiterhin dazu ausgebildet ist, eine Zeitspanne mit einem Startzeitpunkt nach einer ersten Referenzzeit als vorbestimmte Zeitspanne vor der Bestimmungszeit und mit einem Endzeitpunkt vor einer zweiten Referenzzeit nach dem Startzeitpunkt und vor einer zusätzlichen vorbestimmten Zeitspanne, aus der Bestimmungszeit als eine stabile Vergleichs-Dosierzeitspanne unter den ersten stabilen Dosierzeitspannen unmittelbar vor der Bestimmungszeit zu bestimmen, und
die Merkmalsgrößen-Analysiereinheit (13) dazu ausgebildet ist, die Vergleichs-Merkmalsgröße aus den in der stabilen Vergleichs-Dosierzeitspanne enthaltenen spezifischen Untersuchungsdaten zu bestimmen.

7. Verarbeitungsvorrichtung nach Anspruch 6,
bei der die Stabile-Dosierzeitspannen-Bestimmungseinheit (12) dazu ausgebildet ist, Zeitspannen-Spezifizierinformation zu erfassen, in der eine Krankheit und die erste Referenzzeit sowie die zweite Referenzzeit miteinander in Beziehung stehen, um die erste Referenzzeit und die zweite Referenzzeit entsprechend einer Zielkrankheit zu erfassen, bei der es sich um eine Krankheit des Patienten handelt, basierend auf der Zeitspannen-Spezifizierinformation, und die stabile Vergleichs-Dosierzeitspanne basierend auf der ersten Referenzzeit und der zweiten Referenzzeit zu bestimmen.

8. Verarbeitungsvorrichtung nach einem der Ansprüche 5 bis 7,
bei der die Merkmalsgrößen-Analysiereinheit (13) dazu ausgebildet ist, die Vergleichs-Merkmalsgröße anhand einer vorbestimmten Anzahl von Stücken der spezifischen Untersuchungsdaten zu erfassen, zeitlich seriell in die Vergangenheit zurückgehend von einem Ende der ersten stabilen Dosierzeitspanne unmittelbar vor der Bestimmungszeit.

9. Verarbeitungsvorrichtung nach einem der Ansprüche 5 bis 8,
bei der die Vergleichs-Merkmalsgröße ein Durchschnittswert mehrerer Stücke der spezifischen Untersuchungsdaten innerhalb der ersten stabilen Dosierzeitspanne entsprechend einem zweiten Untersuchungsgegenstand als einem vorbestimmten Untersuchungsgegenstand ist.

10. Verarbeitungsvorrichtung nach einem der Ansprüche 5 bis 8,
bei der die Vergleichs-Merkmalsgröße ein Wert einer Approximationskurve entsprechend der Bestimmungszeit in der Approximationskurve ist, berechnet aus mehreren Stücken der spezifischen Untersuchungsdaten innerhalb der ersten stabilen Dosierzeitspanne entsprechend einem dritten Untersuchungsgegenstand als einem vorbestimmten Untersuchungsgegenstand.

11. Verarbeitungsvorrichtung nach einem der Ansprüche 5 bis 9,
bei der unter den ersten Bestimmungsbedingungen bestimmt wird, dass es eine Anormalität zu der vorbestimmten Zeit für den Fall gibt, dass eine Differenz zwischen der Vergleichs-Merkmalsgröße und der ersten Bestimmungs-Merkmalsgröße, oder ein Verhältnis zwischen der Vergleichs-Merkmalsgröße und der ersten Bestimmungs-Merkmalsgröße erste Schwellenwertbedingungen nicht erfüllt.

12. Verarbeitungsvorrichtung nach Anspruch 11,
bei der unter den ersten Schwellenwertbedingungen eine Vergleichs-Merkmalsgröße für Statistiken, das ist eine Merkmalsgröße entsprechend der Vergleichs-Merkmalsgröße und eine erste Bestimmungs-Merkmalsgröße für Statistiken entsprechend der ersten Bestimmungs-Merkmalsgröße, berechnet werden anhand mehrerer Stücke von Untersuchungsdaten mehrerer Vergleichspatienten in der Vergangenheit, mehrerer Bestimmungsparameter für Statistiken entsprechend einem Bestimmungsparameter, für die eine Schwellenwertbestimmung unter den ersten Bestimmungsbedingungen erfolgt, anhand der berechneten Vergleichs-Merkmalsgröße für Statistiken und der ersten Bestimmungs-Merkmalsgröße für Statistiken berechnet werden, und ein Wert, der von einem Durchschnittswert der mehreren berechneten Bestimmungsparameter für Statistiken um einen Wert gleich oder größer als ein Schwankungsbereich der Bestimmungsparameter für Statistiken entfernt ist, als ein Schwellenwert eingestellt wird.

13. Verarbeitungsvorrichtung nach einem der Ansprüche 5 bis 12,
bei der für den Fall, dass ein Wert der spezifischen Untersuchungsdaten innerhalb der ersten stabilen Dosierzeitspanne in einem vorbestimmten Normalbereich liegt, die Merkmalsgrößen-Analysiereinheit (13) dazu ausgebildet ist, die Vergleichs-Merkmalsgröße dadurch zu berechnen, dass der Wert der spezifischen Untersuchungsdaten durch eine Obergrenze und eine Untergrenze des Normalbereichs ersetzt wird.

14. Verarbeitungsvorrichtung nach einem der Ansprüche 1 und 3 bis 13,
bei der die Stabile-Dosierzeitspannen-Bestimmungseinheit (12) dazu ausgebildet ist, eine zweite stabile Dosierzeitspanne zu bestimmen, das ist eine stabile Dosierzeitspanne einschließlich der Bestimmungszeit in einem Endstadium,
die Merkmalsgrößen-Analysiereinheit (13) dazu ausgebildet ist, eine zweite Merkmalsgröße aus mehreren Stücken von Untersuchungsdaten des Patienten innerhalb der zweiten stabilen Dosierzeitspanne zu erfassen, und
der Bestimmungsabschnitt (14) weiterhin einen zweiten Bestimmungsabschnitt (14B) enthält, um zu bestimmen, ob zweite Bestimmungsbedingungen zum Bestimmen des Vorhandenseins oder des Fehlens einer Anormalität zu der Bestimmungszeit basierend auf der zweiten Bestimmungs-Merkmalsgröße erfüllt sind.

15. Verarbeitungsvorrichtung nach Anspruch 14,
bei der die zweite Bestimmungs-Merkmalsgröße ein Koeffizient einer Approximationskurve ist, berechnet aus mehreren Stücken der spezifischen Untersuchungsdaten innerhalb der zweiten stabilen Dosierzeitspanne entsprechend einem vierten Untersuchungsgegenstand, der ein vorbestimmter Untersuchungsgegenstand ist.

16. Verarbeitungsvorrichtung nach Anspruch 15,
bei der unter den zweiten Bestimmungsbedingungen festgestellt wird, dass es eine Anormalität zu der Bestimmungszeit gibt, falls die zweite Bestimmungs-Merkmalsgröße zweite Schwellenwertbedingungen nicht erfüllt.

17. Verarbeitungsvorrichtung nach einem der Ansprüche 14 bis 16,
bei der der Bestimmungsabschnitt (14) ausgebildet ist zum Klassifizieren der zweiten Bestimmungs-Merkmalsgröße gemäß einem von einem Verschlechterungstrend, einem Verbesserungstrend und weiteren Trends, basierend auf der zweiten Bestimmungs-Merkmalsgröße,
der zweite Bestimmungsabschnitt (14B) ausgebildet ist zum Bestimmen, ob die zweiten Bestimmungsbedingungen erfüllt sind, falls die zweite Bestimmungs-Merkmalsgröße ein Verschlechterungstrend ist,
der erste Bestimmungsabschnitt (14A) ausgebildet ist zum Bestimmen, ob die ersten Bestimmungsbedingungen erfüllt sind, falls die zweite Bestimmungs-Merkmalsgröße ein Verbesserungstrend ist,
der erste Bestimmungsabschnitt (14A) ausgebildet ist zum Bestimmen, ob die zusätzlichen ersten Bestimmungsbedingungen erfüllt sind, falls die zweite Bestimmungs-Merkmalsgröße anderen Trends entspricht,
die ersten Bestimmungsbedingungen Bedingungen sind, unter denen die Vergleichs-Merkmalsgröße eine Bestimmungszeit-Vergleichs-Merkmalsgröße ist, das ist ein Wert einer Approximationskurve entsprechend der Bestimmungszeit, die erhalten wird auf der Grundlage der aus mehreren Stücken der Untersuchungsdaten entsprechend einem fünften Untersuchungsgegenstand als vorbestimmtem Untersuchungsgegenstand innerhalb der ersten stabilen Dosierzeitspanne berechneten Approximationskurve, und unter denen festgestellt wird, dass es eine Anormalität gibt, falls eine Differenz zwischen der Bestimmungszeit-Vergleichs-Merkmalsgröße und der ersten Bestimmungs-Merkmalsgröße oder ein Verhältnis zwischen der Bestimmungszeit-Vergleichsmerkmalsgröße und der ersten Bestimmungs-Merkmalsgröße dritte Schwellenwertbedingungen nicht erfüllt, und
die zusätzlichen ersten Bestimmungsbedingungen Bedingungen sind, unter denen die Vergleichs-Merkmalsgröße ein Durchschnittswert mehrerer Stücke der Untersuchungsdaten entsprechend einem sechsten Untersuchungsgegenstand als vorbestimmtem Untersuchungsgegenstand innerhalb der ersten stabilen Dosierzeitspanne ist, und unter denen festgestellt wird, dass es eine Anormalität zu der Bestimmungszeit für den Fall gibt, dass eine Differenz zwischen dem Durchschnittswert und der ersten Bestimmungs-Merkmalsgröße oder ein Verhältnis zwischen dem Durchschnittswert und der ersten Bestimmungs-Merkmalsgröße vierte Schwellenwertbedingungen nicht erfüllt.

18. Verarbeitungsvorrichtung nach einem der Ansprüche 1 bis 17, weiterhin umfassend:
eine zweite Anzeigesteuereinheit (15B), ausgebildet zum Anzeigen einer stabilen Dosierzeitspanne auf einem Anzeigebildschirm,
wobei die Stabile-Dosierzeitspannen-Bestimmungseinheit (12) ausgebildet ist zum Empfangen und Erfassen einer Eingabe zum Ändern der angezeigten stabilen Dosierzeitspanne, und die stabile Dosierzeitspanne ändert.

19. Verarbeitungsvorrichtung nach einem der Ansprüche 2 bis 18,
der die Dosierangaben-Erfassungseinheit (11) dazu ausgebildet ist, Arznei-Spezifizierinformation zu erfassen, wobei jede Krankheit zu einer mit der Krankheit verbundenen Arznei gehört, von der bekannt ist, dass sie für diese Krankheit verabreicht wird, und zum Auswählen und zum Erfassen der Dosierangaben entsprechend der zu der Arznei gehörigen Krankheit entsprechend der Zielkrankheit, basierend auf der erfassten Arznei-Spezifizierinformation.

20. Verarbeitungsvorrichtung nach einem der Ansprüche 6 bis 19,
bei der der Bestimmungsabschnitt (14) weiterhin einen dritten Bestimmungsabschnitt enthält, um zu bestimmen, ob dritte Bestimmungsbedingungen zum Bestimmen des Vorhandenseins oder des Fehlens einer Anormalität zu der Bestimmungszeit erfüllt sind oder nicht, basierend auf einer Differenz zwischen einem Wert der Untersuchungsdaten entsprechend einem siebten Untersuchungsgegenstand als vorbestimmten Untersuchungsgegenstand zu der Bestimmungszeit, und einem Wert von Untersuchungsdaten entsprechend dem siebten Untersuchungsgegenstand unmittelbar vor der Bestimmungszeit, oder basierend auf einem Verhältnis zwischen einem Wert der Untersuchungsdaten entsprechend einem achten Untersuchungsgegenstand als vorbestimmtem Untersuchungsgegenstand zu der Bestimmungszeit und einem Wert von Untersuchungsdaten entsprechend dem achten Untersuchungsgegenstand unmittelbar vor der Bestimmungszeit, und
in dem Bestimmungsabschnitt (14) der dritte Bestimmungsabschnitt dazu ausgebildet ist, zu bestimmen, ob die dritten Bestimmungsbedingungen erfüllt sind oder nicht, und der erste Bestimmungsabschnitt dazu ausgebildet ist, das Vorhandensein oder das Fehlen einer Anormalität zu der Bestimmungszeit gemäß den ersten Bestimmungsbedingungen nur in dem Fall zu bestimmen, dass anhand der dritten Bestimmungsbedingungen festgestellt wird, dass es keine Anormalität gibt.

21. Verarbeitungsvorrichtung nach einem der Ansprüche 14 bis 19,
bei der der Bestimmungsabschnitt (14) weiterhin einen dritten Bestimmungsabschnitt enthält, um zu bestimmen, ob dritte Bestimmungsbedingungen zum Bestimmen des Vorhandenseins oder des Fehlens einer Anormalität zu der Bestimmungszeit erfüllt sind, basieren auf einer Differenz zwischen einem Wert der Untersuchungsdaten entsprechend einem neunten Untersuchungsgegenstand als vorbestimmtem Untersuchungsgegenstand zu der Bestimmungszeit, und einem Wert von Untersuchungsdaten entsprechend dem neunten Untersuchungsgegenstand unmittelbar vor der Bestimmungszeit, oder basierend auf einem Verhältnis zwischen einem Wert der Untersuchungsdaten entsprechend einem neunten Untersuchungsgegenstand als vorbestimmtem Untersuchungsgegenstand und der vorbestimmten Zeit, und einem Wert von Untersuchungsdaten entsprechend dem neunten Untersuchungsgegenstand unmittelbar vor der Bestimmungszeit, und
in dem Bestimmungsabschnitt (14) der dritte Bestimmungsabschnitt dazu ausgebildet ist, zu bestimmen, ob die dritten Bestimmungsbedingungen erfüllt sind oder nicht, und der zweite Bestimmungsabschnitt ausgebildet ist zum Bestimmen des Vorhandenseins oder des Fehlens einer Anormalität zu der Bestimmungszeit abhängig von den zweiten Bestimmungsbedingungen nur dann, wenn basierend auf den dritten Bestimmungsbedingungen festgestellt wird, dass es keine Anormalität gibt.

22. Verarbeitungsverfahren für medizinische Information, ausgeführt von einer Verarbeitungsvorrichtung für medizinische Information, umfassend:
einen Dosierangaben-Erfassungsschritt des Erfassens mehrerer Dosierangaben, in denen Information, die eine einem Patienten zu verabreichende Arznei, eine Arzneimenge und ein Dosierdatum der Arznei miteinander in Beziehung stehen; und
einen Stabile-Dosierzeitspannen-Bestimmungsschritt des Identifizierens einer Dosierzeitspanne gleich oder länger als eine vorbestimmte Zeitspanne gemäß einer Zielkrankheit und einem vorbestimmten Zweck unter Dosierzeitspannen der Arznei, für die angenommen wird, dass sowohl die arzneispezifizierende Information als auch die Arzneimenge gleich sind, basierend auf mehreren erfassten Dosierangaben, des Spezifizierens der identifizierten Dosierzeitspanne als gemeinsame Dosierangabenzeitspanne, und des Bestimmens einer Zeitspanne als stabile Dosierzeitspanne, welche eine vorbestimmte Anfangs- oder Endzeitspanne aus der spezifizierten gemeinsamen Dosierangabenzeitspanne ausschließt,
Erfassen medizinischer Information einschließlich eines Gegenstands von Untersuchungen, die an dem Patienten vorgenommen werden, Untersuchungsdaten des Patienten entsprechend jedem Untersuchungsgegenstand, und einer Untersuchungszeit der Untersuchungsdaten; und
Extrahieren der medizinischen Information entsprechend dem Untersuchungsgegenstand, der zu der Untersuchungszeit während der stabilen Dosierzeitspanne vorgenommen wird, aus der erfassten medizinischen Information als spezifische medizinische Information, und Erfassen einer Merkmalsgröße aus spezifischen Untersuchungsdaten, bei denen es sich um die Untersuchungsdaten entsprechend der extrahierten spezifischen medizinischen Information handelt,
Erfassen einer Bestimmungszeit des Patienten, und Bestimmen des Vorhandenseins oder des Fehlens einer Anormalität in Bezug auf den Bestimmungszweck der Zielkrankheit zu der Bestimmungszeit nach Maßgabe von Bestimmungsbedingungen entsprechend dem Bestimmungszweck der Zielkrankheit, basierend auf der aus den spezifischen Untersuchungsdaten extrahierten Merkmalsgröße, die in der stabilen Dosierzeitspanne vor der Bestimmungszeit enthalten ist.

23. Computerlesbarer Aufzeichnungsträger, auf den ein Verarbeitungsprogramm für medizinische Information gespeichert ist, um einen Computer zu veranlassen, die Schritte nach Anspruch 22 auszuführen.

## Revendications

1. Dispositif de traitement d'informations médicales, comprenant :
une unité d'acquisition de détails de dosage (11) configurée pour acquérir une pluralité de détails de dosage, où des informations spécifiant un médicament administré à un patient sujet, une quantité du médicament, et une date de dosage du médicament sont associées les unes aux autres, et
une unité de détermination de période de dosage stable (12) configurée pour identifier une période de dosage supérieure ou égale à une période prédéterminée conformément à une maladie cible et un but de détermination, parmi des périodes de dosage du médicament sur lesquelles il est considéré qu'à la fois les informations spécifiant le médicament et la quantité du médicament sont identiques, sur la base de la pluralité de détails de dosage acquis, pour spécifier la période de dosage identifiée comme une période commune de détails de dosage, et pour déterminer une période excluant une période initiale ou finale prédéterminée à partir de la période commune de détails de dosage spécifiée comme une période de dosage stable ;
une unité d'acquisition d'informations médicales (16) configurée pour acquérir des informations médicales incluant un élément parmi des inspections réalisées sur le patient sujet, des données d'inspection du patient sujet correspondant à chaque élément d'inspection, et un temps d'inspection des données d'inspection, et
une unité d'analyse de quantité de caractéristique (13) configurée pour extraire les informations médicales correspondant à l'élément d'inspection, lequel est réalisé au temps d'inspection durant la période de dosage stable, à partir des informations médicales acquises, comme informations médicales spécifiques, et pour acquérir une quantité de caractéristique parmi des données d'inspection spécifiques, lesquelles sont les données d'inspection correspondant aux informations médicales spécifiques extraites,
dans lequel l'unité d'analyse de quantité de caractéristique (13) inclut en outre une section de détermination configurée pour acquérir un temps de détermination du patient sujet, et pour déterminer la présence ou l'absence d'une anormalité par rapport au but de détermination de la maladie cible au temps de détermination conformément à des conditions de détermination correspondant au but de détermination de la maladie cible sur la base de la quantité de caractéristique extraite des données d'inspection spécifiques incluses dans la période de dosage stable avant le temps de détermination.

2. Dispositif de traitement d'informations médicales selon la revendication 1,
dans lequel l'unité d'acquisition de détails de dosage (11) est apte à acquérir une maladie du patient sujet comme la maladie cible, et à sélectionner et acquérir les détails de dosage correspondant à un médicament associé à une maladie, lequel est connu comme étant administré pour la maladie cible, parmi les détails de dosage du patient sujet.

3. Dispositif de traitement d'informations médicales selon la revendication 1,
dans lequel l'unité d'analyse de quantité de caractéristique (13) est apte à acquérir des informations de détermination, où une maladie, un premier élément d'inspection, lequel est un élément d'inspection prédéterminé correspondant à la maladie, des informations de spécification de quantité de caractéristique, lesquelles sont des informations spécifiant la quantité de caractéristique à partir des données d'inspection correspondant au premier élément d'inspection, et les conditions de détermination correspondant à ces informations de spécification de quantité de caractéristique, sont associés les uns aux autres, et à acquérir la quantité de caractéristique conformément aux informations de spécification de quantité de caractéristique correspondant à la maladie du patient sujet sur la base des informations de détermination, et
la section de détermination (14) est apte à déterminer la présence ou l'absence d'une anormalité au temps de détermination conformément aux conditions de détermination correspondant aux informations de spécification de quantité de caractéristique sur la base des informations de détermination acquises.

4. Dispositif de traitement d'informations médicales selon la revendication 3, comprenant en outre :
une première unité de commande d'affichage (15A) pour afficher au moins un morceau d'informations inclus dans les informations de détermination correspondant à la détermination, sur un écran d'affichage, dans un cas où il est déterminé qu'il existe une anormalité au temps de détermination sur la base des informations de détermination.

5. Dispositif de traitement d'informations médicales selon l'une quelconque des revendications 1, 3 et 4,
dans lequel l'unité de détermination de période de dosage stable (12) est apte à déterminer une première période de dosage stable immédiatement avant le temps de détermination parmi des premières périodes de dosage stable, lesquelles sont les périodes de dosage stable avant le temps de détermination ;
l'unité d'analyse de quantité de caractéristique est apte à acquérir une quantité de caractéristique de comparaison, laquelle est la quantité de caractéristique déterminée à partir des données d'inspection spécifiques de la première période de dosage stable immédiatement avant le temps de détermination, et une première quantité de caractéristique de détermination, laquelle est une quantité de caractéristique correspondant à la quantité de caractéristique de comparaison déterminée à partir des données d'inspection du patient sujet au temps de détermination, et
la section de détermination (14) inclut une première section de détermination (14A) pour déterminer si oui ou non les premières conditions de détermination pour déterminer la présence ou l'absence d'une anormalité au temps de détermination sur la base de la quantité de caractéristique de comparaison et de la première quantité de caractéristique de détermination sont satisfaites.

6. Dispositif de traitement d'informations médicales selon la revendication 5,
dans lequel l'unité de détermination de période de dosage stable (12) est en outre apte à déterminer une période présentant un temps de début après un premier temps de référence, lequel est une période prédéterminée avant le temps de détermination, et présentant un temps de fin avant un second temps de référence, lequel est après le temps de début et avant une période prédéterminée supplémentaire à partir du temps de détermination, parmi les premières périodes de dosage stable immédiatement avant le temps de détermination, comme une période de dosage stable de comparaison, et
l'unité d'analyse de quantité de caractéristique (13) est apte à déterminer la quantité de caractéristique de comparaison à partir des données d'inspection spécifiques incluses dans la période de dosage stable de comparaison.

7. Dispositif de traitement d'informations médicales selon la revendication 6,
dans lequel l'unité de détermination de période de dosage stable (12) est apte à acquérir des informations de spécification de période, où une maladie et le premier temps de référence et le second temps de référence sont associés les uns aux autres, à acquérir le premier temps de référence et le second temps de référence correspondant à une maladie cible, laquelle est une maladie du patient sujet, sur la base des informations de spécification de période, et à déterminer la période de dosage stable de comparaison sur la base du premier temps de référence et du second temps de référence.

8. Dispositif de traitement d'informations médicales selon l'une quelconque des revendications 5 à 7,
dans lequel l'unité d'analyse de quantité de caractéristique (13) est apte à acquérir la quantité de caractéristique de comparaison à l'aide d'un nombre prédéterminé de morceaux de données d'inspection spécifiques remontant dans un passé suivant des séries chronologiques à partir d'une fin de la première période de dosage stable immédiatement avant le temps de détermination.

9. Dispositif de traitement d'informations médicales selon l'une quelconque des revendications 5 à 8,
dans lequel la quantité de caractéristique de comparaison est une valeur moyenne d'une pluralité de morceaux des données d'inspection spécifiques dans la première période de dosage stable correspondant à un deuxième élément d'inspection, lequel est un élément d'inspection prédéterminé.

10. Dispositif de traitement d'informations médicales selon l'une quelconque des revendications 5 à 8,
dans lequel la quantité de caractéristique de comparaison est une valeur d'une courbe approximative correspondant au temps de détermination dans la courbe approximative calculée à partir d'une pluralité de morceaux des données d'inspection spécifiques dans la première période de dosage stable correspondant à un troisième élément d'inspection, lequel est un élément d'inspection prédéterminé.

11. Dispositif de traitement d'informations médicales selon l'une quelconque des revendications 5 à 9,
dans lequel dans les premières conditions de détermination, il est déterminé qu'il existe une anormalité au temps de détermination dans un cas où une différence entre la quantité de caractéristique de comparaison et la première quantité de caractéristique de détermination ou un rapport entre la quantité de caractéristique de comparaison et la première quantité de caractéristique de détermination ne satisfait pas des premières conditions de valeur seuil.

12. Dispositif de traitement d'informations médicales selon la revendication 11,
dans lequel dans les premières conditions de valeur seuil, une quantité de caractéristique de comparaison pour statistiques, laquelle est une quantité de caractéristique correspondant à la quantité de caractéristique de comparaison, et une première quantité de caractéristique de détermination pour statistiques correspondant à la première quantité de caractéristique de détermination sont calculées à l'aide d'une pluralité de morceaux des données d'inspection d'une pluralité de sujets de comparaison dans un passé ; une pluralité de paramètres de détermination pour statistiques correspondant à un paramètre de détermination, pour lequel une détermination de valeur seuil dans les premières conditions de détermination est réalisée, sont calculés à l'aide de la quantité de caractéristique de comparaison calculée pour statistiques, et d'une première quantité de caractéristique de détermination pour statistiques, et une valeur éloignée d'une valeur moyenne de la pluralité de paramètres de détermination calculés pour statistiques à raison d'une valeur supérieure ou égale à une plage de variation des paramètres de détermination pour statistiques est réglée comme une valeur seuil.

13. Dispositif de traitement d'informations médicales selon l'une quelconque des revendications 5 à 12,
dans lequel dans un cas où une valeur des données d'inspection spécifiques dans la première période de dosage stable est comprise dans une plage normale prédéterminée, l'unité d'analyse de quantité de caractéristique (13) est apte à calculer la quantité de caractéristique de comparaison en remplaçant la valeur des données d'inspection spécifique par une limite supérieure ou une limite inférieure de la plage normale.

14. Dispositif de traitement d'informations médicales selon l'une quelconque des revendications 1, et 3 à 13,
dans lequel l'unité de détermination de période de dosage stable (12) est apte à déterminer une seconde période de dosage stable, laquelle est la période de dosage stable incluant le temps de détermination lors d'un stade final ;
l'unité d'analyse de quantité de caractéristique (13) est apte à acquérir une seconde quantité de détermination à partir d'une pluralité de morceaux des données d'inspection du patient sujet dans la seconde période de dosage stable, et
la section de détermination (14) inclut une deuxième section de détermination (14B) pour déterminer si oui ou non des deuxièmes conditions de détermination pour déterminer la présence ou l'absence d'une anormalité au temps de détermination sur la base de la seconde quantité de caractéristique de détermination sont satisfaites.

15. Dispositif de traitement d'informations médicales selon la revendication 14,
dans lequel la seconde quantité de caractéristique de détermination est un coefficient d'une courbe approximative calculée à partir d'une pluralité de morceaux des données d'inspection spécifiques dans la seconde période de dosage stable correspondant à un quatrième élément d'inspection, lequel est un élément d'inspection prédéterminé.

16. Dispositif de traitement d'informations médicales selon la revendication 15,
dans lequel dans les deuxièmes conditions de détermination, il est déterminé qu'il existe une anormalité au temps de détermination dans un cas où la seconde quantité de caractéristique de détermination ne satisfait pas des deuxièmes conditions de valeur seuil.

17. Dispositif de traitement d'informations médicales selon l'une quelconque des revendications 14 à 16,
dans lequel la section de détermination (14) est apte à classer la seconde quantité de caractéristique de détermination suivant l'une quelconque de tendances parmi une tendance de détérioration, une tendance d'amélioration, et d'autres tendances sur la base de la seconde quantité de caractéristique de détermination ;
la deuxième section de détermination (14B) est apte à déterminer si oui ou non les deuxièmes conditions de détermination sont satisfaites dans un cas où la seconde quantité de caractéristique de détermination est une tendance de détérioration ;
la première section de détermination (14A) est apte à déterminer si oui ou non les premières conditions de détermination sont satisfaites dans un cas où la seconde quantité de caractéristique de détermination est une tendance d'amélioration ;
la première section de détermination (14A) est apte à déterminer si oui ou non les premières conditions de détermination supplémentaires sont satisfaites dans un cas où la seconde quantité de caractéristique de détermination est d'autres tendances ;
les premières conditions de détermination sont des conditions où la quantité de caractéristique de comparaison est une quantité de caractéristique de comparaison de temps de détermination, laquelle est une valeur d'une courbe approximative correspondant au temps de détermination, lequel est obtenu sur la base de la courbe approximative calculée à partir d'une pluralité de morceaux des données d'inspection correspondant à un cinquième élément d'inspection, lequel est un élément d'inspection prédéterminé dans la première période de dosage stable, et où il est déterminé qu'il existe une anormalité dans un cas où une différence entre la quantité de caractéristique de comparaison de temps de détermination et la première quantité de caractéristique de détermination ou un rapport entre la quantité de caractéristique de comparaison de temps de détermination et la première quantité de caractéristique de détermination ne satisfait pas des troisièmes conditions de valeur seuil, et
les premières conditions de détermination supplémentaires sont des conditions où la quantité de caractéristique de comparaison est une valeur moyenne d'une pluralité de morceaux des données d'inspection correspondant à un sixième élément d'inspection, lequel est un élément d'inspection prédéterminé dans la première période de dosage stable, et où il est déterminé qu'il existe une anormalité au temps de détermination dans un cas où une différence entre la valeur moyenne et la première quantité de caractéristique de détermination ou un rapport entre la valeur moyenne et la première quantité de caractéristique de détermination ne satisfait pas des quatrièmes conditions de valeur seuil.

18. Dispositif de traitement d'informations médicales selon l'une quelconque des revendications 1 à 17, comprenant en outre :
une deuxième unité de commande d'affichage (15B) apte à afficher la période de dosage stable sur un écran d'affichage, et
dans lequel l'unité de détermination de période de dosage stable (12) est apte à recevoir et acquérir une entrée afin de modifier la période de dosage stable affichée, et modifie la période de dosage stable.

19. Dispositif de traitement d'informations médicales selon l'une quelconque des revendications 2 à 18,
dans lequel l'unité d'acquisition de détails de dosage (11) est apte à acquérir des informations spécifiant un médicament, où chaque maladie est associée au médicament associé à une maladie, lequel est connu comme étant administré pour la maladie, et à sélectionner et acquérir les détails de dosage correspondant au médicament associé à une maladie correspondant à la maladie cible sur la base des informations de spécification de médicament acquises.

20. Dispositif de traitement d'informations médicales selon l'une quelconque des revendications 6 à 19,
dans lequel la section de détermination (14) inclut en outre une troisième section de détermination pour déterminer si oui ou non des troisièmes conditions de détermination pour déterminer la présence ou l'absence d'une anormalité au temps de détermination sur la base d'une différence entre une valeur des données d'inspection correspondant à un septième élément d'inspection, lequel est un élément d'inspection prédéterminé au temps de détermination, et une valeur des données d'inspection correspondant au septième élément d'inspection immédiatement avant le temps de détermination, ou d'un rapport entre une valeur des données d'inspection correspondant à un huitième élément d'inspection, lequel est un élément d'inspection prédéterminé au temps de détermination, et une valeur des données d'inspection correspondant au huitième élément d'inspection immédiatement avant le temps de détermination sont satisfaites, et
dans la section de détermination (14), la troisième section de détermination est apte à déterminer si oui ou non les troisièmes conditions de détermination sont satisfaites, et la première section de détermination est apte à déterminer la présence ou l'absence d'une anormalité au temps de détermination conformément aux premières conditions de détermination uniquement dans un cas où il est déterminé qu'il n'existe aucune anormalité sur la base des troisièmes conditions de détermination.

21. Dispositif de traitement d'informations médicales selon l'une quelconque des revendications 14 à 19,
dans lequel la section de détermination (14) inclut en outre une troisième section de détermination pour déterminer si oui ou non des troisièmes conditions de détermination pour déterminer la présence ou l'absence d'une anormalité au temps de détermination sur la base d'une différence entre une valeur des données d'inspection correspondant à un neuvième élément d'inspection, lequel est un élément d'inspection prédéterminé au temps de détermination, et une valeur des données d'inspection correspondant au neuvième élément d'inspection, immédiatement avant le temps de détermination, ou d'un rapport entre une valeur des données d'inspection correspondant à un neuvième élément d'inspection, lequel est un élément d'inspection prédéterminé au temps de détermination, et une valeur des données d'inspection correspondant au neuvième élément d'inspection immédiatement avant le temps de détermination sont satisfaites, et
dans la section de détermination (14), la troisième section de détermination est apte à déterminer si oui ou non les troisièmes conditions de détermination sont satisfaites, et la deuxième section de détermination est apte à déterminer la présence ou l'absence d'une anormalité au temps de détermination conformément aux deuxièmes conditions de détermination uniquement dans un cas où il est déterminé qu'il n'existe aucune anormalité sur la base des troisièmes conditions de détermination.

22. Procédé de traitement d'informations médicales exécuté par un dispositif de traitement d'informations médicales comprenant :
une étape d'acquisition de détails de dosage pour acquérir une pluralité de détails de dosage, où des informations spécifiant un médicament administré à un patient sujet, une quantité du médicament, et une date de dosage du médicament sont associées les unes aux autres ;
une étape de détermination de période de dosage stable pour identifier une période de dosage supérieure ou égale à une période prédéterminée conformément à une maladie cible et un but de détermination, parmi des périodes de dosage du médicament, sur lesquelles il est considéré qu'à la fois les informations spécifiant le médicament et la quantité du médicament sont identiques, sur la base de la pluralité de détails de dosage acquis, pour spécifier la période de dosage identifiée comme une période commune de détails de dosage, et pour déterminer une période excluant une période initiale ou finale prédéterminée à partir de la période commune de détails de dosage spécifiée comme une période de dosage stable ;
une étape pour acquérir des informations médicales incluant un élément parmi des inspections réalisées sur le patient sujet, des données d'inspection du patient sujet correspondant à chaque élément d'inspection, et un temps d'inspection des données d'inspection ;
une étape pour extraire des informations médicales correspondant à l'élément d'inspection, lequel est réalisé au temps d'inspection durant la période de dosage stable, à partir des informations médicales acquises, comme informations médicales spécifiques, et pour acquérir une quantité de caractéristique parmi des données d'inspection spécifiques, lesquelles sont les données d'inspection correspondant aux informations médicales spécifiques extraites, et
une étape pour acquérir un temps de détermination du patient sujet, et pour déterminer la présence ou l'absence d'une anormalité par rapport au but de détermination de la maladie cible au temps de détermination conformément à des conditions de détermination correspondant au but de détermination de la maladie cible sur la base de la quantité de caractéristique extraite des données d'inspection spécifiques incluses dans la période de dosage stable avant le temps de détermination.

23. Support d'enregistrement lisible par ordinateur sur lequel est stocké un programme de traitement d'informations médicales faisant en sorte qu'un ordinateur exécute les étapes selon la revendication 22.
